# EUROPEAN PATENT APPLICATION

(11) **EP 4 671 365 A1**
(43) Date of publication of application: **31.12.2025**
(21) Application number: 24759754.5
(22) Date of filing: 23.02.2024
(51) Int. Cl.: C12N 5/0783, C12N 5/10, A61K 35/17, A61P 35/00

(54) **KIT FOR INDUCING STEM CELLS TO DIFFERENTIATE INTO NK CELLS, USE OF KIT, AND USE OF COMBINATION OF NK CELLS AND TMZ IN TREATMENT FOR TMZ DRUG-RESISTANT GBM**

(30) Priority: 24.02.2023 CN 202310166354; 01.09.2023 CN 202311121831
(71) Applicant: Xellsmart Biomedical (Suzhou) Co., Ltd, Suzhou, Jiangsu 210008 (CN)
(72) Inventor: LI, Xiang, Shanghai 201111 (CN)
(74) Representative: Schnappauf, Georg
(86) International application number: PCT/CN2024/078236
(87) International publication number: WO 2024/175080

(57) **Abstract**

The present invention relates to the field of biotechnology, and provides a kit and method for inducing stem cells to differentiate into natural killer (NK) cells, and a use of the kit. In the kit, a culture medium having clear chemical components, chemical small molecules, and cytokines are used. The kit does not involve substances such as serum which are easy to cause unstable factors such as batch differences, is cheaper in price than a known commercial differentiation culture medium, and reduces the differentiation period. The present invention further provides a pharmaceutical composition and method for treating glioblastoma (GBM), particularly drug-resistant GBM, and a use of the pharmaceutical composition. Specifically, the present invention relates to treatment for GBM by combining immune cells and a chemotherapy drug. More specifically, the present invention relates to synergistic treatment for TMZ drug-resistant GBM by combining NK cells and TMZ.

## Description

### TECHNICAL FIELD

The present disclosure relates to the field of biological technology, and particularly, to a kit for inducing the differentiation of stem cells into natural killer cells and use thereof. The present disclosure further relates to the combination treatment for a solid tumor with an immune cell and a chemotherapeutic agent, and particularly, to the synergistic treatment of TMZ-resistant GBM with a combination of an NK cell and TMZ.

### BACKGROUND

Natural killer cells are part of the innate immune system and play an important role in immune surveillance, host defense against viral infections, and antagonizing tumor cells. Recent studies have also shown that NK cells are a potential therapeutic target for cancer treatment. In the human body, NK cells account for 8-20% of the lymphocytes. Unlike other lymphocytes (including B cells, T cells, and natural killer T (NKT) cells), NK cells do not express antigen-specific receptors such as clonal B cell receptors, T cell receptor/CD3ε complexes, or the like, but work in an antigen-independent manner (which usually does not form immunological memory or long-term protective immunity). The cytotoxicity of NK cells is regulated by means of activation and inhibitory receptors on the surface of same. Unlike T cells, NK cells retain their natural and innate ability to recognize receptors and target tumor cells; compared with T cells, NK cells do not require strict HLA (human leukocyte antigen) matching, giving rise to a lower risk of GVHD (graft-versus-host disease). Moreover, in multiple known clinical trials, they exhibited no safety issues such as cytokine release syndrome in T cell therapy, thereby providing possibilities of manufacturing "ready-to-use" allogeneic cell therapy products. Peripheral blood- or umbilical cord blood-derived NK cells are subject to limited donors and sources, and the obtained cells are inconsistent in the state, quality, and yield. In addition, T cell contamination may occur during the cell expansion. Moreover, the expanded NK cells may deteriorate after cryopreservation. However, existing methods for inducing the differentiation of stem cells into natural killer cells are problematic. The method for producing NK cells derived from iPS cell differentiation involves the use of MEK inhibitors, GSK3 inhibitors, ROCK inhibitors, and the like, but features a low efficiency in differentiation into NK cells and limited NK cell yield. The method for producing NK cells derived from iPS cell differentiation in Patent No. CN107429230A is as follows: The differentiation into hematopoietic progenitor cells is induced in a 3D embryoid body form, and the CD34⁺/CD45⁺ cells are enriched and then induced to differentiate into NK cells. The method uses heterogeneous substances such as commercial media with uncertain ingredients, serum, and the like, involves complex operation processes, and is unfavorable to subsequent preparation of clinical-grade cell products. The method for deriving NK cells from iPS cell differentiation in Patent No. CN112608895A is as follows: The differentiation into permanent hematopoietic progenitor cells (HPCs) is induced in embryoid bodies (EBs), then the EBs are seeded into a culture vessel coated with a matrix protein to give permanent HPCs, and finally, the differentiation into NK cells is induced. The method features a low efficiency in acquiring permanent HPCs, and the differentiation into NK cells is readily affected by the cell state of HPCs and the efficiency of differentiation into HPCs; in addition, the cells should be seeded in a petri dish containing a matrix protein that may also affect the differentiation into NK cells. The method for deriving NK cells from iPS cell differentiation in Patent No. CN113801846A is as follows: Human induced pluripotent stem cells are seeded into a wave bioreactor and a cell factory to give endothelial progenitor cells, and the endothelial progenitor cells are induced to differentiate into NK progenitor cells, which further differentiate into NK cells. In the differentiation system, the potency of endothelial progenitor cells for differentiation into various endothelial cells increases the risk that the efficiency of the differentiation into NK cells may be affected by external environments (e.g., parameter settings of the bioreactor).

Cerebral glioma is a malignant tumor forming in the brain or in nerves that originate from the brain. Cerebral gliomas are pathologically graded I-IV by the World Health Organization (WHO), among which grade IV is glioblastoma (GBM). GBM is the most common and aggressive central nervous system tumor found within the cranial cavity. At present, the therapeutic effects of available standard treatments against glioblastoma are far from ideal with poor prognoses and a 5-year overall survival rate of only 6.8% (Ostrom QT, et al. CBTRUS Statistical Report: Primary Brain and Other Central Nervous System Tumors Diagnosed in the United States in 2012-2016. Neuro Oncol. 2019 Nov 1;21(Suppl 5):v1-v100). For the treatment of primary GBM at the first diagnosis, available standard treatments include tumor resection at maximal safety level and the combination of radiotherapy (RT) with temozolomide (TMZ) chemotherapy.

However, by the response to TMZ, GBMs may be classified into sensitive and resistant types. Patients suffering from sensitive GBMs exhibit a better prognosis for the TMZ standard treatment. However, virtually all GBMs may relapse, with the median overall survival OS being only 6 to 9 months from the progression or relapse. The underlying mechanism is that sensitive GBM tumor cells may develop various strategies to escape TMZ effects, convert into resistant GBMs, and finally behave as the primary resistant GBMs with resistance to TMZ, recurrence, metastasis, and life-threatening potential (Weller M, et al. Standards of care for treatment of recurrent glioblastoma--are we there yet? Neuro Oncol. 2013 Jan;15(1):4-27). Therefore, new therapeutic strategies for treating TMZ-resistant GBMs are urgently needed.

Immunotherapy is a novel therapy antagonizing tumors with adoptive immune cells or the innate immune system, and is changing the treatment modalities for various cancers. However, to date, breakthroughs of immunotherapy have not been achieved in the treatment of GBMs due to reasons of high GBM heterogeneity, target inhomogeneity, low utilization rate of protein drugs due to the blood-brain barrier, and the like. Novel therapeutic strategies for improving the treatment of TMZ-resistant GBMs using adoptive immune cells in combination with TMZ are needed in the art.

### SUMMARY

In a first aspect, the present disclosure provides a kit, comprising a second culture medium, a fourth culture medium, and a fifth culture medium, wherein the second culture medium is a basal medium comprising a BMP signaling pathway activator; the fourth culture medium is a basal medium comprising at least one, at least two, at least three, or at least four of the following: a BMP signaling pathway activator, a vascular endothelial growth factor, a colony-stimulating factor, and an interleukin; the fifth culture medium is a basal medium comprising at least one, at least two, at least three, at least four, at least five, or at least six of the following: a BMP signaling pathway activator, a vascular endothelial growth factor, a colony-stimulating factor, an interleukin, a stem cell agonist, and a stem cell expansion agent.

In one embodiment, the kit further comprises a third culture medium, wherein the third culture medium is a basal medium comprising at least one, at least two, or at least three of the following: a BMP signaling pathway activator, a growth factor, and a TGFβ/ALK inhibitor.

In one embodiment, the kit further comprises a first culture medium, wherein the first culture medium is a basal medium comprising a ROCK inhibitor.

In one embodiment, the second culture medium is a basal medium comprising a BMP signaling pathway activator and a GSK3 inhibitor.

In a second aspect, the present disclosure provides use of the kit according to the first aspect of the present disclosure in preparing an embryoid body, preparing a mesodermal cell, preparing a hemogenic endothelial cell, preparing a natural killer (NK) cell, preparing a product for inducing the differentiation of a stem cell into an embryoid body, preparing a product for inducing the differentiation of a stem cell into a mesodermal cell, preparing a product for inducing the differentiation of a stem cell into a hemogenic endothelial cell, and/or preparing a product for inducing the differentiation of a stem cell into an NK cell.

In a third aspect, the present disclosure provides a method for inducing the differentiation of a stem cell into a natural killer (NK) cell, comprising: using the kit according to the first aspect of the present disclosure.

In one embodiment, the method comprises: step (1), culturing a stem cell to give an embryoid body; step (2), subjecting the embryoid body to mesoderm differentiation culture to give a mesodermal cell; step (3), subjecting the mesodermal cell to hemogenic endothelium differentiation culture to give a hemogenic endothelial cell; and step (4), culturing the hemogenic endothelial cell in the fourth culture medium and the fifth culture medium of the kit according to the first aspect of the present disclosure to give the NK cell.

In a fourth aspect, the present disclosure provides a natural killer (NK) cell, prepared using the kit according to the first aspect of the present disclosure and/or the method according to the third aspect of the present disclosure.

In a fifth aspect, the present disclosure provides a pharmaceutical composition, comprising the NK cell according to the fourth aspect of the present disclosure.

In a sixth aspect, the present disclosure provides use of the NK cell according to the fourth aspect of the present disclosure or the pharmaceutical composition according to the fifth aspect of the present disclosure in preparing a medicament for preventing and/or treating a tumor.

In a seventh aspect, the present disclosure provides a pharmaceutical combination for use in treating glioblastoma, comprising an active ingredient, wherein the active ingredient comprises a natural killer (NK) cell and a chemotherapeutic agent; the chemotherapeutic agent comprises temozolomide (TMZ).

In one embodiment, the glioblastoma is TMZ-resistant.

In one embodiment, the NK cell is an unmodified NK cell.

In one embodiment, the NK cell includes, but is not limited to, an NK-92 cell, a peripheral blood- or umbilical cord blood-derived NK cell, and an induced pluripotent stem cell (iPSC)- or embryonic stem cell-derived NK cell.

In one embodiment, the NK cell is an induced pluripotent stem cell (iPSC)-derived NK cell. In one embodiment, the NK cell is an unmodified, induced pluripotent stem cell-derived NK cell.

In one embodiment, the induced pluripotent stem cell (iPSC)-derived NK cell is prepared using the kit according to the first aspect of the present disclosure or the method according to the third aspect of the present disclosure.

In one embodiment, the induced pluripotent stem cell (iPSC)-derived NK cell is prepared using the method for inducing the differentiation of a stem cell into a natural killer cell described herein. In one embodiment, the pharmaceutical combination further comprises an additional chemotherapeutic agent selected from an anti-cancer agent, a metabolic antagonist, a DNA demethylating agent, a plant-derived anti-tumor agent, an alkylating agent, an antimetabolite, an anti-cancer antibiotic, a topoisomerase inhibitor, a mitotic inhibitor, a differentiation agent, and a hormonal therapeutic agent.

In one embodiment, the additional chemotherapeutic agent is selected from carmustine, lomustine, vorinostat, mebendazole, imatinib mesylate, AG119, and any therapeutic derivative thereof.

In one embodiment, the active ingredient does not comprise trimetrexate.

In one embodiment, the active ingredient does not comprise an immune checkpoint inhibitor. In one embodiment, the pharmaceutical combination is a kit.

In an eighth aspect, the present disclosure further provides use of the pharmaceutical combination according to the seventh aspect of the present disclosure in preparing a medicament for treating glioblastoma.

In one embodiment, the chemotherapeutic agent is administered before, after, or concurrent with the administration of the NK cell.

In one embodiment, the chemotherapeutic agent is administered 8 to 72 h before, 8 to 72 h after, or 8 to 72 h from the administration of the NK cell.

### BRIEF DESCRIPTION OF THE DRAWINGS

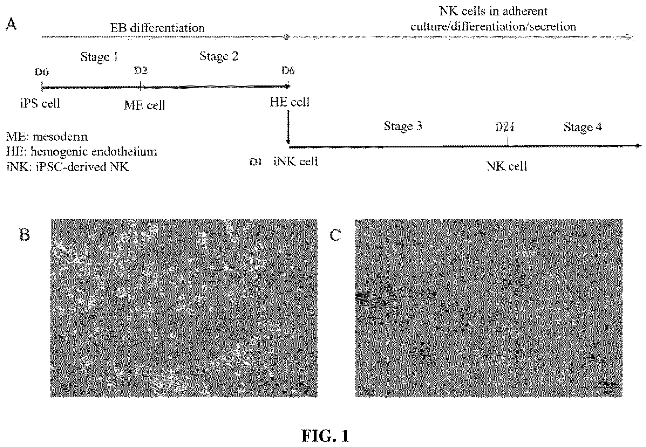

FIG. 1 shows the schematic of the induction of the differentiation of human induced pluripotent stem cells into natural killer cells and the cell differentiation images in Example 2, in which A shows the schematic of the induction of the differentiation of human induced pluripotent stem cells into natural killer cells in Example 2; B shows the cell morphology image (observed under a 4 × objective lens) on day 2 of the NK cell differentiation culture of the hemogenic endothelial cells (iNK-D2, EB adherent culture D2) in Example 2; and C shows the cell morphology image (observed under a 10 × objective lens) on day 6 of the NK cell differentiation culture of the hemogenic endothelial cells (iNK-D20) in Example 2.

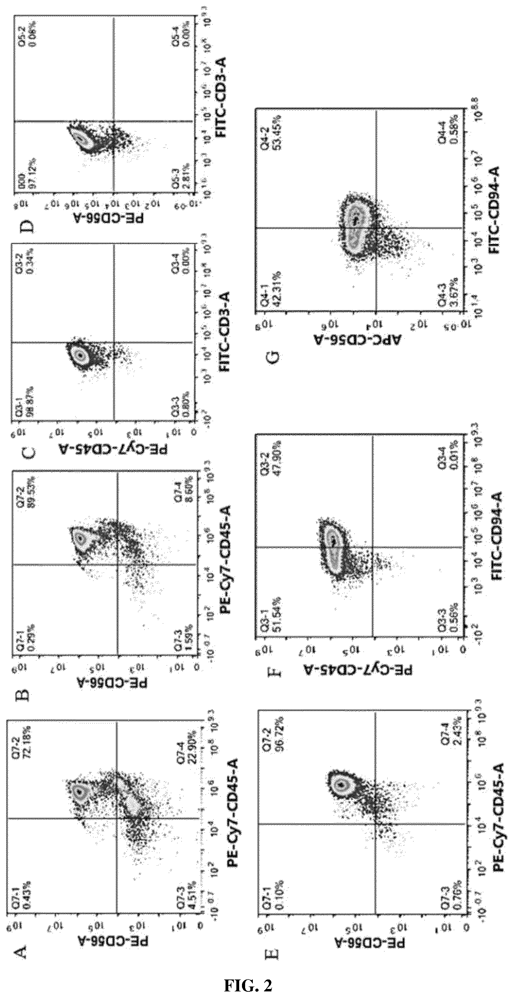

FIG. 2 shows the flow cytometric diagrams of the NK cells collected on days 13, 16, and 20 of the NK cell differentiation culture of the hemogenic endothelial cells in Example 2, in which A shows the assay results of the CD45⁺/CD56⁺ cells among the NK cells collected on day 13 of the NK cell differentiation culture of the hemogenic endothelial cells in Example 2; B shows the assay results of the CD45⁺/CD56⁺ cells among the NK cells collected on day 16 of the NK cell differentiation culture of the hemogenic endothelial cells in Example 2; C shows the assay results of the CD45⁺/CD3⁻ cells among the NK cells collected on day 20 of the NK cell differentiation culture of the hemogenic endothelial cells in Example 2; D shows the assay results of the CD56⁺/CD3⁻ cells among the NK cells collected on day 20 of the NK cell differentiation culture of the hemogenic endothelial cells in Example 2; E shows the assay results of the CD45⁺/CD56⁺ cells among the NK cells collected on day 20 of the NK cell differentiation culture of the hemogenic endothelial cells in Example 2; F shows the assay results of the CD45⁺/CD94⁺ cells among the NK cells collected on day 20 of the NK cell differentiation culture of the hemogenic endothelial cells in Example 2; G shows the assay results of the CD56⁺/CD94⁺ cells among the NK cells collected on day 20 of the NK cell differentiation culture of the hemogenic endothelial cells in Example 2.

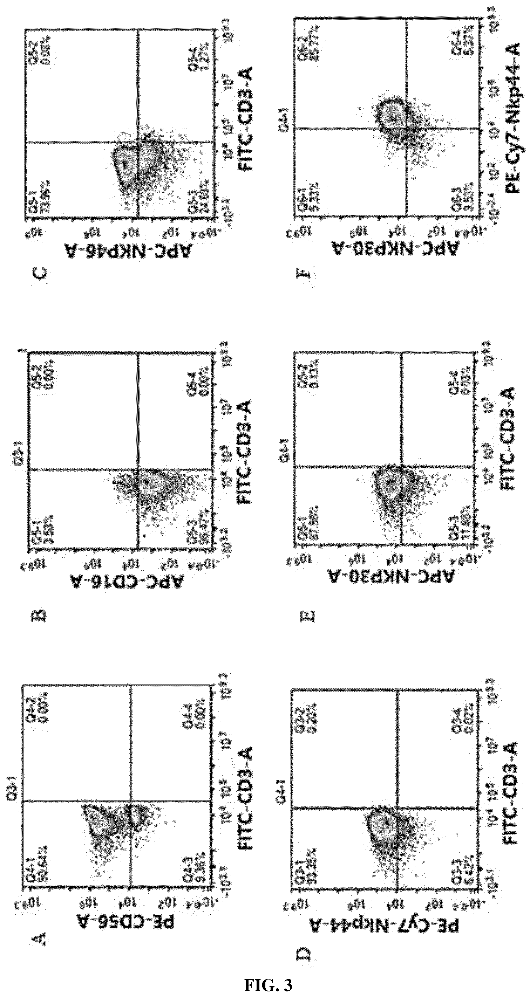

FIG. 3 shows the flow cytometric diagrams of the NK cells collected on day 20 of the NK cell differentiation culture of the hemogenic endothelial cells in Example 2, in which A shows the assay results of the CD3⁻/CD45⁺/CD56⁺ cells among the NK cells collected on day 20 of the NK cell differentiation culture of the hemogenic endothelial cells in Example 2; B shows the assay results of the CD3⁻/CD16⁻ cells among the NK cells collected on day 20 of the NK cell differentiation culture of the hemogenic endothelial cells in Example 2; C shows the assay results of the CD3⁻/Nkp46⁺ cells among the NK cells collected on day 20 of the NK cell differentiation culture of the hemogenic endothelial cells in Example 2; D shows the assay results of the CD3⁻/CD56⁺/Nkp44⁺ cells among the NK cells collected on day 20 of the NK cell differentiation culture of the hemogenic endothelial cells in Example 2; E shows the assay results of the CD3⁻/CD56⁺/Nkp30⁺ cells among the NK cells collected on day 20 of the NK cell differentiation culture of the hemogenic endothelial cells in Example 2; F shows the assay results of the CD3⁻/CD56⁺/Nkp44⁺/Nkp30⁺ cells among the NK cells collected on day 20 of the NK cell differentiation culture of the hemogenic endothelial cells in Example 2.

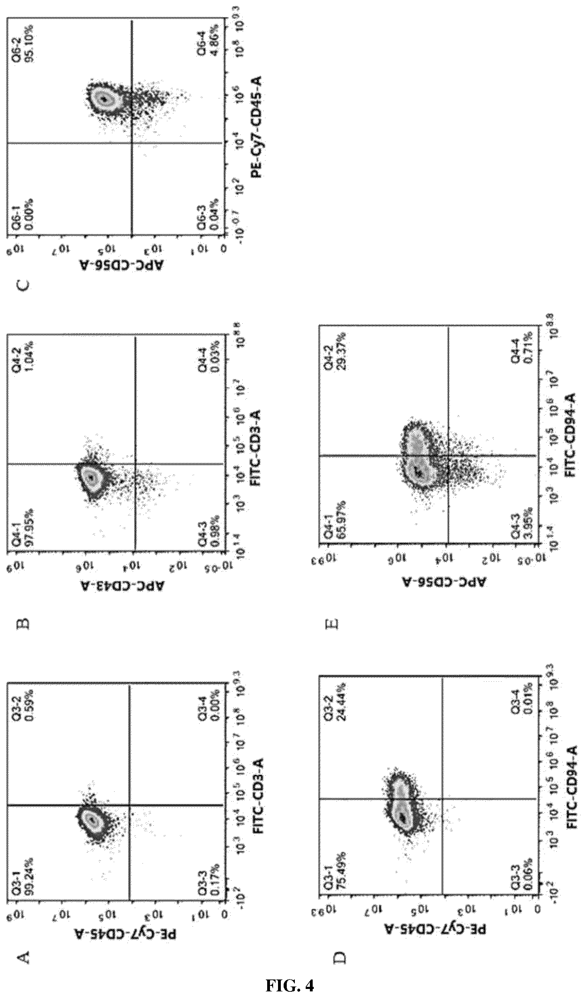

FIG. 4 shows the flow cytometric diagrams of the NK cells collected on day 20 of the NK cell differentiation culture of the hemogenic endothelial cells in Comparative Example 2, in which A shows the assay results of the CD45⁺/CD3⁻ cells among the NK cells collected on day 20 of the NK cell differentiation culture of the hemogenic endothelial cells in Comparative Example 2; B shows the assay results of the CD43⁺/CD3⁻ cells among the NK cells collected on day 20 of the NK cell differentiation culture of the hemogenic endothelial cells in Comparative Example 2; C shows the assay results of the CD45⁺/CD56⁺ cells among the NK cells collected on day 20 of the NK cell differentiation culture of the hemogenic endothelial cells in Comparative Example 2; D shows the assay results of the CD45⁺/CD94⁺ cells among the NK cells collected on day 20 of the NK cell differentiation culture of the hemogenic endothelial cells in Comparative Example 2; E shows the assay results of the CD56⁺/CD94⁺ cells among the NK cells collected on day 20 of the NK cell differentiation culture of the hemogenic endothelial cells in Comparative Example 2.

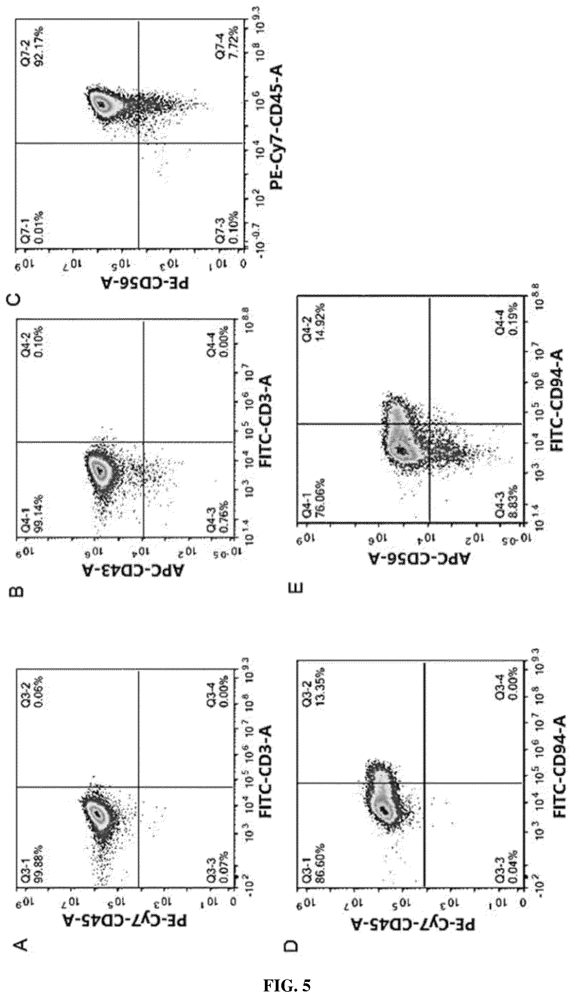

FIG. 5 shows the flow cytometric diagrams of the NK cells collected on day 20 of the NK cell differentiation culture of the hemogenic endothelial cells in Comparative Example 4, in which A shows the assay results of the CD45⁺/CD3⁻ cells among the NK cells collected on day 20 of the NK cell differentiation culture of the hemogenic endothelial cells in Comparative Example 4; B shows the assay results of the CD43⁺/CD3⁻ cells among the NK cells collected on day 20 of the NK cell differentiation culture of the hemogenic endothelial cells in Comparative Example 4; C shows the assay results of the CD45⁺/CD56⁺ cells among the NK cells collected on day 20 of the NK cell differentiation culture of the hemogenic endothelial cells in Comparative Example 4; D shows the assay results of the CD45⁺/CD94⁺ cells among the NK cells collected on day 20 of the NK cell differentiation culture of the hemogenic endothelial cells in Comparative Example 4; E shows the assay results of the CD56⁺/CD94⁺ cells among the NK cells collected on day 20 of the NK cell differentiation culture of the hemogenic endothelial cells in Comparative Example 4.

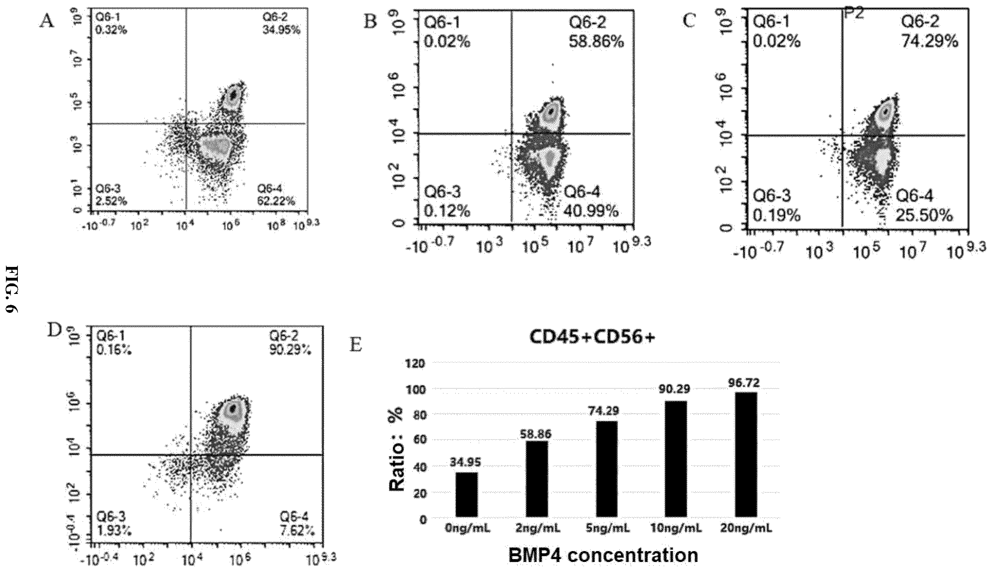

FIG. 6 shows the flow cytometric diagrams of the NK cells collected on day 20 of the NK cell differentiation culture of the hemogenic endothelial cells in Comparative Example 6, Example 4-1, Example 4-3, Example 4-2, and Example 2, in which A shows the flow cytometry results of the NK cells collected on day 20 of the NK cell differentiation culture of the hemogenic endothelial cells in Comparative Example 6; B shows the flow cytometry results of the NK cells collected on day 20 of the NK cell differentiation culture of the hemogenic endothelial cells in Example 4-1; C shows the flow cytometry results of the NK cells collected on day 20 of the NK cell differentiation culture of the hemogenic endothelial cells in Example 4-3; D shows the flow cytometry results of the NK cells collected on day 20 of the NK cell differentiation culture of the hemogenic endothelial cells in Example 4-2; E shows the bar chart of the proportion of the CD45⁺/CD56⁺ cells to the NK cells collected on day 20 of the NK cell differentiation culture of the hemogenic endothelial cells in Comparative Example 6, Example 4-1, Example 4-3, Example 4-2, and Example 2.

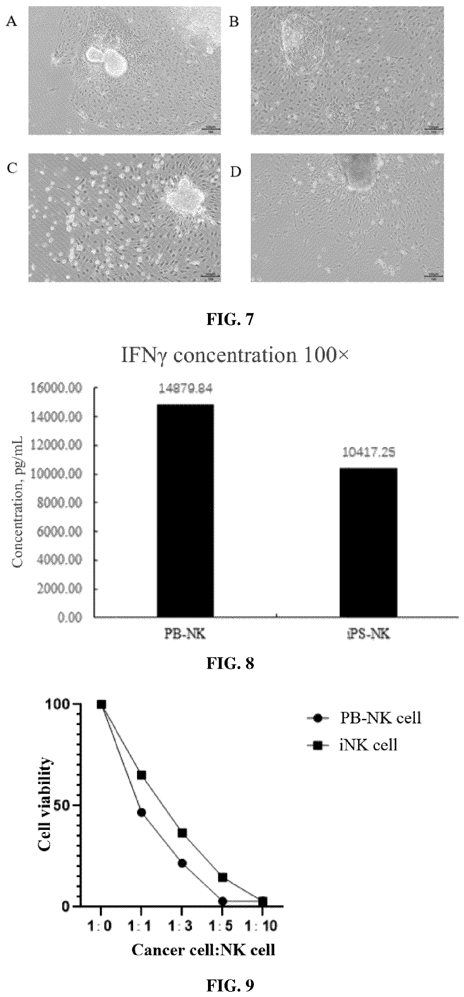

FIG. 7 shows the bright field images of the NK cells collected on day 20 of the NK cell differentiation culture of the hemogenic endothelial cells in Comparative Example 6, Example 4-1, Example 4-3, and Example 4-2, in which A shows the bright field image of the NK cells collected on day 20 of the NK cell differentiation culture of the hemogenic endothelial cells in Comparative Example 6; B shows the bright field image of the NK cells collected on day 20 of the NK cell differentiation culture of the hemogenic endothelial cells in Example 4-1; C shows the bright field image of the NK cells collected on day 20 of the NK cell differentiation culture of the hemogenic endothelial cells in Example 4-3; D shows the bright field image of the NK cells collected on day 20 of the NK cell differentiation culture of the hemogenic endothelial cells in Example 4-2.
FIG. 8 shows the IFN-γ expression results of the NK cells in Example 2 and the peripheral blood-derived NK (PB-NK) cells treated in the same condition.
FIG. 9 shows the results of the killing effect assay of the NK cells in Example 2 and the peripheral blood-derived NK (PB-NK) cells treated in the same condition on Hela cells.
FIG. 10 shows the schematic flow chart of the *in vivo* NK cell killing assay in Effect Example 4.
FIG. 11 shows the results of the *in vivo* NK cell killing assay in Effect Example 4.
FIG. 12 shows the expansion folds of the differentiation of different batches of NK cells in Effect Example 5.
FIG. 13 shows the results of cell viability comparison before and after NK cell cryopreservation in Effect Example 5.
FIGs. 14A-D show the sensitivity response of different GBM cell strains to TMZ.
FIGs. 15A and B show the effect of TMZ on cell cycle arrest in different GBM cell strains.
FIG. 16 shows the induced up-regulation of the expression of various immune ligands after 48 hours of TMZ treatment in resistant GBM tumor cells.
FIG. 17 shows the characterization results of NK cells derived from *in vitro* directed induction of differentiation of iPSCs.
FIGs. 18A and B show the degranulation activity assay results of iPSC-NK in combination with TMZ in killing GBM.
FIG. 19 shows the killing activity of iPSC-NK in combination with TMZ against resistant GBM (T98G).
FIG. 20 shows the characterization results of the NK92 cell strain and PB-NK cells.
FIG. 21 shows the killing activity of NK cells derived from different sources in combination with TMZ against resistant GBM.

### DETAILED DESCRIPTION

### General Definitions and Terms

All the patents, patent applications, scientific publications, instructions and guidelines of manufacturers, and the like cited herein, whether above-mentioned or below-described, are herein incorporated by reference in their entirety. Any content herein should not be construed as an admission that the present disclosure is not entitled to antedate such disclosures.

Unless otherwise specified, the scientific and technical terms used herein have the meanings generally understood by those skilled in the art. Moreover, the terms associated with the protein and nucleic acid chemistry, molecular biology, cell and tissue culture, and microbiology used herein are the terms widely used in the corresponding fields (see, e.g., Molecular Cloning: A Laboratory Manual, 2nd Edition, J. Sambrook et al. eds., Cold Spring Harbor Laboratory Press, Cold Spring Harbor 1989). Meanwhile, in order to better understand the present disclosure, the definitions and explanations of the relevant terms are provided below.

As used herein, the expressions "include", "comprise", "contain", and "have" are open-ended and mean to include the listed elements, steps, or components but not to exclude other unlisted elements, steps, or components. The expression "consist of..." excludes any element, step, or component that is not designated. The expression "consist essentially of..." means that the scope is limited to the designated elements, steps, or components, in addition to elements, steps, or components that are optionally present and do not significantly affect the essential and novel characteristics of the claimed subject matter. It will be appreciated that the expressions "consist essentially of..." and "consist of..." are encompassed within the meaning of the expression "comprise".

As used herein, unless otherwise specified in the context, the expression in singular form "a", "an", or "the" includes plural referents. The term "one or more" or "at least one" encompasses 1, 2, 3, 4, 5, 6, 7, 8, 9, or more.

The enumeration of numerical ranges herein merely serves as a simplified representation for individually referencing each different value falling within the range. Unless otherwise specified herein, each individual value is incorporated into this specification as if it were individually enumerated herein. Unless the contrary is specifically indicated, the numerical values or ranges shown herein are all modified by "about" to represent the enumerated or claimed numerical value or range ±20%, ±10%, ±5%, or ±3%.

The term "pluripotent cell" refers to a cell that is capable of self-renewing and proliferating while remaining in an undifferentiated state, and capable of differentiating into a specialized cell by induction in appropriate conditions.

As used herein, the term "pluripotent stem cells" refers to the potential of differentiating into any one of the following three germ layers: endoderm (e.g., gastric junction, gastrointestinal tract, lung, etc.), mesoderm (e.g., muscle, bone, blood, genitourinary tissue, etc.), or ectoderm (e.g., epidermal tissue and nervous system tissue). As used herein, the term "pluripotent stem cell" also includes "induced pluripotent stem cell" or "iPSC", which is a pluripotent stem cell derived from a non-pluripotent cell and constructed by reprogramming a non-pluripotent cell via introducing specific transcription factors. Exemplary human pluripotent stem cell lines include the H1 human pluripotent stem cell line and the H9 human pluripotent stem cell line. Other exemplary pluripotent stem cell lines include those available from the National Institutes of Health HumanEmbryonic Stem Cell Registry and Howard Hughes Medical Institute HUES collection (as described in Cowan CA, et al. Derivation of embryonic stem-cell lines from human blastocysts. N Engl J Med. 2004 Mar 25;350(13):1353-6). Pluripotent stem cells can differentiate into different cells by induction, and can be used for different prophylactic or therapeutic purposes to prevent or treat different diseases. As will be appreciated by those skilled in the art, the differentiation method depends on the needed cell type involving the application of known techniques. For example, the cells may be differentiated in suspension and then formulated in a gel matrix form, such as a matrigel, gelatin, or fibrin/thrombin form, to promote cell viability. Generally, the differentiation can be determined by evaluating the presence of cell-specific markers, as known in the art. For example, in certain differentiation conditions, a cell may be differentiated into a cardiomyocyte, a nerve cell, a glial cell, an endothelial cell, a T cell, an NK cell, an NKT cell, a macrophage, a hematopoietic progenitor cell, a mesenchymal cell, an islet cell, a chondrocyte, a retinal pigment epithelial cell, a renal cell, a hepatocyte, a thyroid cell, a skin cell, a blood cell, or an epithelial cell. In some embodiments, an iPSC is differentiated into an NK cell.

The iPSC-derived NK cell is also referred to as an iNK cell herein. Herein, the terms "iNK cell", "iPSC-NK cell", and "induced pluripotent stem cell (iPSC)-derived NK cell" are used interchangeably.

As used herein, the term "totipotent" refers to the ability of a cell to form a whole organism. For example, in mammals, only the fertilized egg and the blastomere during the first cleavage are totipotent. In some embodiments, the pluripotent stem cell described herein does not have totipotency and cannot form a whole organism.

A cell may be derived from, e.g., a human or non-human mammal. Exemplary non-human mammals include, but are not limited to, mice, rats, cats, dogs, rabbits, guinea pigs, hamsters, sheep, pigs, horses, cows, and non-human primates. In some embodiments, the cell is derived from an adult human or a non-human mammal. In some embodiments, the cell is derived from a neonatal human, an adult human, or a non-human mammal.

As used herein, the term "subject" or "patient" refers to any animal; e.g., a domesticated animal, a zoo animal, or a human. The "subject" or "patient" may be a mammal, such as a dog, a cat, a bird, livestock, or a human. Specific examples of "subject" and "patient" include, but are not limited to, an individual (particularly a human) suffering from a disease or disorder associated with the liver, heart, lung, kidney, pancreas, brain, neural tissue, blood, bone, bone marrow, etc. In some embodiments, the "subject" and "patient" refer to an individual (particularly a human) suffering from glioblastoma. In some preferred embodiments, the "subject" and "patient" refer to an individual (particularly a human) suffering from TMZ-resistant glioblastoma.

As used herein, the terms "reduce" and "decrease" are both generally used to denote a decrease by a statistically significant amount. However, for the avoidance of doubt, the "reduce" or "decrease" includes a decrease by at least 10% as compared to a reference level, such as a decrease by at least about 20%, or at least about 30%, or at least about 40%, or at least about 50%, or at least about 60%, or at least about 70%, or at least about 80%, or at least about 90%, or up to and including a 100% decrease (i.e., a level indicating absence as compared to a reference sample), or any decrease between 10% and 100%, as compared to a reference level. As used herein, the term "increase" is generally used to denote an increase by a statistically significant amount; for the avoidance of any doubt, the term "increase" refers to an increase by at least 10% as compared to a reference level, such as an increase by at least about 20%, or at least about 30%, or at least about 40%, or at least about 50%, or at least about 60%, or at least about 70%, or at least about 80%, or at least about 90%, or up to and including a 100% increase, or any increase between 10% and 100%, compared to a reference level; or by at least about 2 folds, or at least about 3 folds, or at least about 4 folds, or at least about 5 folds; or at least about 10 folds, or any increase between 2 folds to 10 folds or greater than 10 folds, as compared to a reference level.

As used herein, the terms "NK cell" and "natural killer cell" are used interchangeably. The NK cells are effector cells of the innate immune system. They belong to the innate lymphoid cell (ILC) family, and are characterized by the presence of surface marker CD56 and the absence of CD3. Most NK cells are found in the peripheral blood, lymph nodes, spleen, and bone marrow. The NK cells are able to protect the host by killing infected, exogenous, stressed, or transformed cells and to kill target cells in the absence of specific antigen stimuli without restrictions corresponding to MHC classes, where the target cells may be tumor cells or virus-carrying cells. Currently, in preclinical and clinical development strategies, the anti-tumor functions of NK cells are enhanced by means of adoptive immunotherapy, direct stimulation, recruitment of NK cells to the tumor microenvironment (TME), blocking inhibitory receptors that limit NK cell functions, and the like. (see, e.g., Bald T, et al. The NK cell-cancer cycle: advances and new challenges in NK cell-based immunotherapies. Nat Immunol. 2020 Aug;21(8):835-847).

As used herein, the term "adoptive cell transfer therapy", "adoptive immune cell therapy", "adoptive immune cell treatment", or "adoptive cell treatment" is an immune cell therapy that modifies and expands immune cells *in vitro* to improve the immunologic functions of the immune cells, and transfuses the immune cells into the patient body to exert the anti-tumor effect. These immune cells may be derived from an allogeneic or autogeneic source. The allogeneic adoptive immunotherapy refers to transferring immune cells derived from an allogeneic (different individual) source to the patient. The autogeneic adoptive immunotherapy refers to the collection, expansion, and transfer of the patient's own immune cells. These immune cells may be natural killer cells, T cells, lymphocytes, or the like.

As used herein, the term "glioblastoma" is the most common primary brain malignancy, accounting for about 57% of gliomas and 48% of primary malignant central nervous system (CNS) tumors (see, e.g., Tan AC, et al. Management of glioblastoma: State of the art and future directions. CA Cancer J Clin. 2020 Jul; 70(4):299-312); problems such as drug resistance, poor patient prognosis, short survival, and the like are present in its treatment. Currently, clinical treatments for GBMs are relatively scant, which mainly include temozolomide (TMZ), carmustine, lomustine, bevacizumab, and the like. Among them, the most extensively used first-line treatment is TMZ in clinical practices. However, by the response to TMZ, GBMs may be classified into sensitive and resistant types. The term "sensitive GBM" refers to a GBM sensitive to the TMZ standard treatment, which has a relatively good prognosis. However, tumors originally sensitive to chemotherapy may gradually develop resistance. Therefore, sensitive GBM tumor cells also develop various strategies to escape TMZ effects and finally convert into resistant GBMs. The term "resistant GBM" refers to a GBM resistant to TMZ and capable of resisting TMZ effects. The drug resistance is not caused by a single factor, but may be associated with various aspects such as the DNA mismatch repairing system, the expression of O6-methylguanine-DNA methyltransferase, the penetration of drugs through the blood-brain barrier (see, e.g., Ortiz R, et al. Temozolomide: An Updated Overview of Resistance Mechanisms, Nanotechnology Advances and Clinical Applications. Curr Neuropharmacol. 2021; 19(4):513-537), and the like.

As used herein, the term "chemotherapeutic agent" refers to a compound or a derivative thereof that is able to mutually interact with cancer cells, thereby reducing the cell proliferation state and/or killing the cells via, e.g., impairing the cell division or DNA synthesis, or effectively targeting rapidly dividing cells by DNA destruction. Examples of a chemotherapeutic agent include, but are not limited to, an anti-cancer agent, a metabolic antagonist, a DNA demethylating agent, a plant-derived anti-tumor agent, an alkylating agent, an antimetabolite, an anti-cancer antibiotic, a topoisomerase inhibitor, a mitotic inhibitor, a differentiation agent, a hormonal therapeutic agent, and a combination of the above agents.

As used herein, the term "alkylating agent" or "alkylation agent" refers to a class of compounds highly active in chemical properties. Such compounds have an active alkyl group, which can be converted into electron-deficient active intermediates. The intermediates form covalent bonds rapidly with many substances with affinity effects in cells. The alkylating agent is able to covalently bind to electron-rich groups (e.g., amino, sulfhydryl, hydroxy, carboxyl, phosphate groups, and the like) in biological macromolecules (e.g., DNAs, RNAs, enzymes, and the like) in cells to render them inactive, break DNA molecules, or cause base mismatch during DNA replication, thereby destroying the structure and functions of the DNA and leading to tumor cell death.

As used herein, the term "temozolomide (TMZ)" is an orally administered alkylating prodrug that can transfer a methyl group to purine bases of DNA (O6-guanine; N7-guanine, and N3-adenine) and is often used in combination with radiotherapy as part of the first-line treatment of malignant gliomas. The chemical structure of TMZ is C₆H₆N₆O₂; the chemical structural formula thereof is shown below; the chemical name is 3,4-dihydro-3-methyl-4-oxoimidazo[5,1-d]-asymmetric-tetrazol-8-amide. The major advantages of TMZ are its high oral bioavailability (almost 100%, although the concentration detected in the cerebrospinal fluid is about 20% of the TMZ plasma concentration) and rapid penetration through the blood-brain barrier due to its lipophilicity and small size (see, e.g., Ortiz R, et al. Temozolomide: An Updated Overview of Resistance Mechanisms, Nanotechnology Advances and Clinical Applications. Curr Neuropharmacol. 2021; 19(4):513-537).

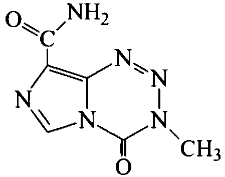

As used herein, administration in any form of "combination", "combination therapy", and/or "combination treatment regimen" refers to at least two therapeutically active drugs or a composition or a pharmaceutical combination comprising at least two therapeutically active ingredients, which can be administered simultaneously in separate or combined formulations, or administered sequentially at different times separated by minutes, hours, or days, while acting together with a given mechanism to provide the desired therapeutic response.

The term "differentiation" refers to a process by which a less specialized cell forms progeny of at least one more specialized new cell type.

The term "embryoid body," or an embryoid or aggregate, refers to a homogeneous or heterogeneous cell cluster comprising differentiated cells, partially differentiated cells, and/or pluripotent stem cells in suspension culture. To summarize some clues inherent to *in vivo* differentiation, certain aspects of the present disclosure may use a three-dimensional embryoid body as an intermediate step. At the start of cell aggregation, differentiation may be initiated and cells may begin to reproduce embryonic development to a limited extent. Despite trophectoderm tissues, they may develop into almost all other types of cells present in an organism. The present disclosure can further promote the differentiation of hematopoietic progenitor cells after the formation of embryoid bodies.

The term "basal medium" refers to a chemically defined medium, including but not limited to basal cell culture media such as Iscove's Modified Dulbecco's Medium (IMDM), Eagle's Basal Medium (BME), MEM, DMEM, Ham's F-12 Medium, RPMI1640 Medium, Fischer's Medium, E8 Medium, mTESR Medium, StemFit Basic 03, StemFit Basic 04, NutriStem hPSC XF Medium, StemMACS iPS Brew Medium, Stem-Partner ACF Medium, TeSR-AOF Medium, TeSR2 Medium, and the like.

The term "multi-colony-stimulating factor" is also known as interleukin-3. It is primarily produced by activated Z cell or T cell clones. It contains 13 amino acid residues, and can serve as an immunomodulator and stimulate the proliferation and differentiation of pluripotent stem cells and various progenitor cells.

The term "stem cell factor" is also known as a mast cell growth factor (MGF), Kit ligand (KL), and Steel factor (SLF). It is an acidic glycoprotein produced by stromal cells in the bone marrow microenvironment, with its glycosyl group attached to the N and O groups of the peptide bond. It has a relative molecular mass of 31000-36000 and consists of two identical subunits non-covalently bound. The isoelectric point PI = 3.8. The SCF has 273 amino acids in total. The signal peptide is from -25 to -1; the extramembrane functional region is from +1 to +189; the transmembrane region is from +190 to +216; and the cytoplasmic functional region is from +217 to +248. Murine and human SCFs are 83% homologous.

The term "vitamin C" includes vitamin C or various forms of salts thereof or various forms of derivatives thereof.

The term "UM171" includes UM171 (CAS number: 1448724-09-1) or various forms of salts thereof or various forms of derivatives thereof.

The term "SR1" includes SR1 (StemRegenin1, CAS number: 1227633-49-9) or various forms of derivatives thereof.

The term "glutamine" refers to L-glutamine, a coded amino acid in protein synthesis and a non-essential amino acid for mammals, serving as an essential supplement for cell culture in the present disclosure.

The term "SB431542" also includes SB431542 and salts thereof, especially pharmaceutically acceptable salts thereof.

The term "CHIR99021" includes CHIR99021 and salts thereof, especially pharmaceutically acceptable salts thereof.

The term "Y-27632" also includes Y-27632 and salts thereof, especially pharmaceutically acceptable salts thereof. A preferred pharmaceutically acceptable salt is Y-27632 ·2HCl.

The term "fibroblast growth factor" refers to a polypeptide secreted by the pituitary and hypothalamus. It can promote fibroblast mitosis and mesodermal cell growth and stimulate angiogenesis, and plays a role in wound healing and limb regeneration. It is divided into acidic (pI 5.6) and basic (pI 9.6) types, i.e., aFGF and bFGF.

The term "bone morphogenetic protein-4" or BMP4 has regulatory effects on the proliferation and differentiation of various cells during embryonic development.

The term "vitamin A-free B27 medium supplement" refers to a customized B-27^{®} medium supplement free of vitamin A. Vitamin A (retinol) can be converted to retinoic acid, which can induce the differentiation of stem cells into nerve cells. Vitamin A-free formulations are ideal for stem cell culture.

### Detection marker for cells

The developmental stages of NK cells are mainly based on the expression levels of CD117, CD45, CD56, and CD94. Increased CD56 expression is pivotal to NK maturation; second to that is CD94/NKG2A expression. Therefore, in the NK differentiation process, the expression of CD45⁺/CD56⁺ is selected to indicate the differentiation stage; the CD3 negative expression is also used to indicate whether T cell interference in the differentiation system is present.

CD45: The cell surface phosphatase CD45 functions in NK cell development and intracellular signal transduction of activated receptors, and is a key enzyme in the induction of NK cell responses.

CD56: also known as neural cell adhesion molecule, a 175- to 185-kDa glycoprotein member of the immunoglobulin superfamily C2 group mainly expressed on human NK cells and a few T lymphocytes mediating MHC. It is expressed on the surface of 95% or more of human NK cells, and is an important surface marker of NK cells. NK cells: In the human body, NK cells are a group of lymphocytes mainly characterized by CD3⁻/CD56⁺.

CD3: a component in the T cell receptor (TCR) complex signaling pathway used to indicate whether T cell interference in the NK differentiation process is present, as part of T cells can also express surface markers of NK cells.

CD94: On the surface of NK cells, CD94 is mainly present in a form homotypic or heterotypic to NKG2A, and is a marker for the maturation of differentiated NK cells.

Nkp30: NKp30 is a member of the CD28 family and the immunoglobulin superfamily (IgSF). It is only expressed on the surface of NK cells as a specific marker of NK cells, and possesses the functionality of activating NK cells, with its ligand being B7-H6. As an NK cell-activating receptor, it plays an important role in the immune surveillance and killing of tumor and pathogen-infected cells by binding to its corresponding ligand.

Nkp44: As a member of the immunoglobulin superfamily (IgSF), NKp44 is only expressed on the surface of activated NK cells as a specific marker for activated NK cells. It has only one V-domain in its extracellular region. The NKp44 cytoplasmic region does not contain ITAM motifs, and the transmembrane region contains positively charged lysine, thereby gaining the function of transducing activation signals by non-covalently binding to DAP12 homodimers containing negatively charged aspartic acid in the transmembrane region and ITAM motifs in the cytoplasmic region. NKp44 is only expressed after NK cell activation and plays a role in further amplifying the signal.

Nkp46: NKp46 is expressed on the surface of all NK cells (including mature, immature, resting, and activated NK cells), and contains 2 Ig-like domains in the extracellular region. NKp46 has a relatively short cytoplasmic domain and positively charged arginine in the transmembrane domain, thereby gaining the function of transducing activation signals by non-covalently binding to CD3ζζ homodimers containing negatively charged aspartic acid in the transmembrane region and ITAM motifs in the cytoplasmic region.

As used herein, the terms "first culture medium" and similarly, "second culture medium", "third culture medium", "fourth culture medium", "fifth culture medium", and the like are used for distinguishing purposes only and are not intended to specify the order in which these media are used. The above terms used in different schemes may be identical or different.

Unless otherwise stated, all methods described herein may be implemented in any suitable order.

### Kit

In a first aspect, the present disclosure provides a kit, comprising a second culture medium, a fourth culture medium, and a fifth culture medium, wherein the second culture medium is a basal medium comprising a BMP signaling pathway activator; the fourth culture medium is a basal medium comprising at least one, at least two, at least three, or at least four of the following: a BMP signaling pathway activator, a vascular endothelial growth factor, a colony-stimulating factor, and an interleukin; the fifth culture medium is a basal medium comprising at least one, at least two, at least three, at least four, at least five, or at least six of the following: a BMP signaling pathway activator, a vascular endothelial growth factor, a colony-stimulating factor, an interleukin, a stem cell agonist, and a stem cell expansion agent.

In some embodiments, the kit is a kit for inducing the differentiation of a stem cell into a natural killer cell.

In one embodiment, the second culture medium is a basal medium comprising a BMP signaling pathway activator and a GSK3 inhibitor.

In one embodiment, the BMP signaling pathway activator of the second culture medium comprises at least one of BMP2, BMP4, SB4, SJ000291942, SJ000063181, SJ000370178, isoliquiritigenin, diosmetin, apigenin, and biochanin. In one preferred embodiment, the BMP signaling pathway activator of the second culture medium comprises BMP4.

In one embodiment, the GSK-3 inhibitor of the second culture medium comprises at least one of a GSK-3α inhibitor and a GSK-3β inhibitor. In one preferred embodiment, the GSK-3 inhibitor of the second culture medium comprises at least one of B216763, TWS119, NP031112, SB216763, CHIR-98014, AZD2858, AZD1080, SB415286, LY2090314, and CHIR-99021. In one more preferred embodiment, the GSK-3 inhibitor of the second culture medium comprises CHIR-99021.

In one embodiment, the concentration of the BMP signaling pathway activator in the second culture medium is 1-100 ng/mL, preferably 2-50 ng/mL, and more preferably 20 ng/mL.

In one embodiment, the concentration of the GSK-3 inhibitor in the second culture medium is 0.1-10 µM, preferably 1-10 µM, and more preferably 3 µM.

In one embodiment, the basal medium of the second culture medium comprises at least one of Iscove's Modified Dulbecco's Medium (IMDM), Eagle's Basal Medium (BME), MEM, DMEM, Ham's F-12 Medium, RPMI1640 Medium, Fischer's Medium, and DMEM/F12 Medium. In one preferred embodiment, the basal medium of the second culture medium comprises RPMI1640 medium.

In one embodiment, the basal medium of the second culture medium comprises at least one, at least two, at least three, or at least four of the following: a non-essential amino acid, glutamine, vitamin C, and a B27 medium supplement. In one preferred embodiment, the basal medium of the second culture medium comprises a non-essential amino acid, glutamine, vitamin C, and a B27 medium supplement.

In one embodiment, the B27 medium supplement is a vitamin A-free B27 medium supplement. In one embodiment, the concentration of the non-essential amino acid in the second culture medium is 0.2%-5% (v/v), preferably 0.5%-2% (v/v), and more preferably 1% (v/v).

In one embodiment, the concentration of glutamine in the second culture medium is 0.2%-5% (v/v), preferably 0.5%-2% (v/v), and more preferably 1% (v/v).

In one embodiment, the concentration of vitamin C in the second culture medium is 10-100 µg/mL, preferably 20-50 µg/mL, and more preferably 50 µg/mL.

In one embodiment, the concentration of the B27 medium supplement in the second culture medium is 1%-10% (v/v), preferably 2% (v/v).

In the present disclosure, the second culture medium is used for inducing the differentiation of an embryoid body into a mesodermal cell.

In one embodiment, the BMP signaling pathway activator of the fourth culture medium and/or the fifth culture medium comprises at least one of BMP2, BMP4, SB4, SJ000291942, SJ000063181, SJ000370178, isoliquiritigenin, diosmetin, apigenin, and biochanin. In one preferred embodiment, the BMP signaling pathway activator of the fourth culture medium and/or the fifth culture medium comprises BMP4.

In one embodiment, the vascular endothelial growth factor of the fourth culture medium and/or the fifth culture medium comprises at least one of VEGF-A, VEGF-165, VEGF-183, VEGF-110, VEGF-121, VEGF-B, VEGF-C, VEGF-D, and VEGF-E. In one preferred embodiment, the vascular endothelial growth factor of the fourth culture medium and/or the fifth culture medium comprises VEGF-165.

In one embodiment, the colony-stimulating factor of the fourth culture medium comprises at least one of G-CSF (granulocyte colony-stimulating factor), M-CSF (macrophage colony-stimulating factor), GM-CSF (recombinant human granulocyte-macrophage colony-stimulating factor), multi-CSF (multi-colony-stimulating factor, also known as IL-3), EPO (erythropoietin), TPO, SCF, and FLT3L. In one preferred embodiment, the colony-stimulating factor of the fourth culture medium comprises at least one of IL-3, SCF, and FLT3L. In one more preferred embodiment, the colony-stimulating factor of the fourth culture medium comprises IL-3, SCF, and FLT3L.

In one embodiment, the colony-stimulating factor of the fifth culture medium comprises at least one of G-CSF (granulocyte colony-stimulating factor), M-CSF (macrophage colony-stimulating factor), GM-CSF (recombinant human granulocyte-macrophage colony-stimulating factor), multi-CSF (multi-colony-stimulating factor, also known as IL-3), EPO (erythropoietin), TPO, SCF, and FLT3L. In one preferred embodiment, the colony-stimulating factor of the fifth culture medium comprises at least one of SCF and FLT3L. In one more preferred embodiment, the colony-stimulating factors of the fifth culture medium comprise SCF and FLT3L.

In one embodiment, the interleukin of the fourth culture medium and/or the fifth culture medium comprises at least one of IL-1, IL-2, IL-6, IL-7, IL-12, IL-15, IL-18, IL-21, and IL-27. In one preferred embodiment, the interleukin of the fourth culture medium and/or the fifth culture medium comprises at least one of IL-7 and IL-15. In one more preferred embodiment, the interleukin of the fourth culture medium and/or the fifth culture medium comprises IL-7 and IL-15.

In one embodiment, the stem cell agonist of the fifth culture medium comprises at least one of UM171 and UM729. In one preferred embodiment, the stem cell agonist comprises UM171. In one embodiment, the stem cell expansion agent of the fifth culture medium comprises at least one of SR1 and PD98059. In one preferred embodiment, the stem cell expansion agent comprises SR1.

In one embodiment, the concentration of the BMP signaling pathway activator in the fourth culture medium is 1-50 ng/mL, preferably 1-10 ng/mL, and more preferably 5 ng/mL.

In one embodiment, the concentration of the vascular endothelial growth factor in the fourth culture medium is 5-50 ng/mL, preferably 5-20 ng/mL, and more preferably 10 ng/mL.

In one embodiment, the concentration of the colony-stimulating factor in the fourth culture medium is 7-200 ng/mL, preferably 16-80 ng/mL, and more preferably 35 ng/mL.

In one embodiment, the concentration of IL-3 in the fourth culture medium is 1-50 ng/mL, preferably 1-10 ng/mL, and more preferably 5 ng/mL.

In one embodiment, the concentration of SCF in the fourth culture medium is 5-100 ng/mL, preferably 10-50 ng/mL, and more preferably 20 ng/mL.

In one embodiment, the concentration of FLT3L in the fourth culture medium is 1-50 ng/mL, preferably 5-20 ng/mL, and more preferably 10 ng/mL.

In one embodiment, the concentration of the interleukin in the fourth culture medium is 2-100 ng/mL, preferably 15-70 ng/mL, and more preferably 30 ng/mL.

In one embodiment, the concentration of IL15 in the fourth culture medium is 1-50 ng/mL, preferably 5-20 ng/mL, and more preferably 10 ng/mL.

In one embodiment, the concentration of IL7 in the fourth culture medium is 1-50 ng/mL, preferably 10-50 ng/mL, and more preferably 20 ng/mL.

In one embodiment, the concentration of the BMP signaling pathway activator in the fifth culture medium is 1-50 ng/mL, preferably 5-10 ng/mL, and more preferably 5 ng/mL.

In one embodiment, the concentration of the vascular endothelial growth factor in the fifth culture medium is 5-50 ng/mL, preferably 5-20 ng/mL, and more preferably 10 ng/mL.

In one embodiment, the concentration of the colony-stimulating factor in the fifth culture medium is 6-150 ng/mL, preferably 15-70 ng/mL, and more preferably 30 ng/mL.

In one embodiment, the concentration of SCF in the fifth culture medium is 5-100 ng/mL, preferably 10-50 ng/mL, and more preferably 20 ng/mL.

In one embodiment, the concentration of FLT3L in the fifth culture medium is 1-50 ng/mL, preferably 5-20 ng/mL, and more preferably 10 ng/mL.

In one embodiment, the concentration of the interleukin in the fifth culture medium is 2-100 ng/mL, preferably 15-70 ng/mL, and more preferably 30 ng/mL.

In one embodiment, the concentration of IL15 in the fifth culture medium is 1-50 ng/mL, preferably 5-20 ng/mL, and more preferably 10 ng/mL.

In one embodiment, the concentration of IL7 in the fifth culture medium is 1-50 ng/mL, preferably 10-50 ng/mL, and more preferably 20 ng/mL.

In one embodiment, the concentration of the stem cell agonist in the fifth culture medium is 0.5-10 µM, preferably 0.5-5 µM, and more preferably 1 µM.

In one embodiment, the concentration of the stem cell expansion agent in the fifth culture medium is 0.5-10 µM, preferably 0.5-5 µM, and more preferably 1 µM.

In one embodiment, the basal medium of the fourth culture medium comprises at least one of Iscove's Modified Dulbecco's Medium (IMDM), Eagle's Basal Medium (BME), MEM, DMEM, Ham's F-12 Medium, RPMI1640 Medium, Fischer's Medium, and DMEM/F12 Medium. In one preferred embodiment, the basal medium of the fourth culture medium comprises Iscove's Modified Dulbecco's Medium (IMDM).

In one embodiment, the basal medium of the fourth culture medium comprises at least one, at least two, at least three, at least four, at least five, or at least six of the following: a non-essential amino acid, glutamine, vitamin C, a B27 medium supplement, and N-acetyl-L-cysteine (NAC). In one preferred embodiment, the basal medium of the fourth culture medium comprises a non-essential amino acid, glutamine, vitamin C, a B27 medium supplement, and N-acetyl-L-cysteine (NAC).

In one embodiment, the B27 medium supplement is a vitamin A-free B27 medium supplement. In one embodiment, the concentration of the non-essential amino acid in the fourth culture medium is 0.2%-5% (v/v), preferably 0.5%-2% (v/v), and more preferably 1% (v/v).

In one embodiment, the concentration of glutamine in the fourth culture medium is 0.2%-5% (v/v), preferably 0.5%-2% (v/v), and more preferably 1% (v/v).

In one embodiment, the concentration of vitamin C in the fourth culture medium is 10-100 µg/mL, preferably 20-50 µg/mL, and more preferably 50 µg/mL.

In one embodiment, the concentration of the B27 medium supplement in the fourth culture medium is 1%-10% (v/v), preferably 2% (v/v).

In one embodiment, the concentration of N-acetyl-L-cysteine in the fourth culture medium is 1-100 µM, preferably 10-50 µM, and more preferably 30 µM.

In one embodiment, the basal medium of the fifth culture medium comprises at least one of Iscove's Modified Dulbecco's Medium (IMDM), Eagle's Basal Medium (BME), MEM, DMEM, Ham's F-12 Medium, RPMI1640 Medium, Fischer's Medium, and DMEM/F12 Medium. In one preferred embodiment, the basal medium of the fifth culture medium comprises Iscove's Modified Dulbecco's Medium (IMDM).

In one embodiment, the basal medium of the fifth culture medium comprises at least one, at least two, at least three, at least four, at least five, or at least six of the following: a non-essential amino acid, glutamine, vitamin C, a B27 medium supplement, and N-acetyl-L-cysteine (NAC). In one preferred embodiment, the basal medium of the fifth culture medium comprises a non-essential amino acid, glutamine, vitamin C, a B27 medium supplement, and N-acetyl-L-cysteine (NAC).

In one embodiment, the B27 medium supplement is a vitamin A-free B27 medium supplement. In one embodiment, the concentration of the non-essential amino acid in the fifth culture medium is 0.2%-5% (v/v), preferably 0.5%-2% (v/v), and more preferably 1% (v/v).

In one embodiment, the concentration of glutamine in the fifth culture medium is 0.2%-5% (v/v), preferably 0.5%-2% (v/v), and more preferably 1% (v/v).

In one embodiment, the concentration of vitamin C in the fifth culture medium is 10-100 µg/mL, preferably 20-50 µg/mL, and more preferably 50 µg/mL.

In one embodiment, the concentration of the B27 medium supplement in the fifth culture medium is 1%-10% (v/v), preferably 2% (v/v).

In one embodiment, the concentration of N-acetyl-L-cysteine in the fifth culture medium is 1-100 µM, preferably 10-50 µM, and more preferably 30 µM.

In the present disclosure, the fourth culture medium and the fifth culture medium are used for inducing the differentiation of a hemogenic endothelial cell into an NK cell and the activation (maturation and/or expansion) of the NK cell.

In one embodiment, the kit further comprises a third culture medium, wherein the third culture medium is a basal medium comprising at least one, at least two, or at least three of the following: a BMP signaling pathway activator, a growth factor, and a TGFβ/ALK inhibitor.

In one embodiment, the BMP signaling pathway activator of the third culture medium comprises at least one of BMP2, BMP4, SB4, SJ000291942, SJ000063181, SJ000370178, isoliquiritigenin, diosmetin, apigenin, and biochanin. In one preferred embodiment, the BMP signaling pathway activator of the third culture medium comprises BMP4.

In one embodiment, the growth factor of the third culture medium comprises at least one of epidermal growth factor (EGF), platelet-derived growth factor (PDGF), fibroblast growth factor (FGF), hepatocyte growth factor (HGF), insulin-like growth factor-I (IGF-1), IGF-II, leukemia inhibitory factor (LIF), nerve growth factor (NGF), oncostatin M (OSM), platelet-derived endothelial cell growth factor (PDECGF), transforming growth factor-α (TGF-α), and vascular endothelial cell growth factor (VEGF). In one preferred embodiment, the growth factor of the third culture medium comprises at least one of fibroblast growth factor (FGF) and vascular endothelial growth factor (VEGF). In one more preferred embodiment, the growth factor of the third culture medium comprises fibroblast growth factor (FGF) and vascular endothelial growth factor (VEGF).

In one embodiment, the vascular endothelial growth factor comprises at least one of VEGF-A, VEGF-165, VEGF-183, VEGF-110, VEGF-121, VEGF-B, VEGF-C, VEGF-D, and VEGF-E. In one preferred embodiment, the vascular endothelial growth factor comprises VEGF-165. In one embodiment, the fibroblast growth factor comprises at least one of an acidic fibroblast factor and a basic fibroblast factor. In one preferred embodiment, the fibroblast growth factor comprises a basic fibroblast factor.

In one embodiment, the TGFβ/ALK inhibitor of the third culture medium comprises at least one of: SB431542, SB-505, A-83-01, GW6604, IN-1130, Ki26894, LY2157299, LY364947 (HTS-466284), LY550410, LY573636, LY580276, NPC-30345, SB-505124, SD-093, Sm16, SM305, SX-007, Antp-Sm2A, and LY2109761. In one preferred embodiment, the TGFβ/ALK inhibitor of the third culture medium comprises SB431542.

In one embodiment, the concentration of the BMP signaling pathway activator in the third culture medium is 1-50 ng/mL, preferably 1-10 ng/mL, and more preferably 5 ng/mL.

In one embodiment, the concentration of the vascular endothelial growth factor in the third culture medium is 1-100 ng/mL, preferably 20-50 ng/mL, and more preferably 50 ng/mL.

In one embodiment, the concentration of the fibroblast growth factor in the third culture medium is 1-100 ng/mL, preferably 5-50 ng/mL, and more preferably 50 ng/mL.

In one embodiment, the concentration of the TGFβ/ALK inhibitor in the third culture medium is 1-50 µM, preferably 5-20 µM, and more preferably 10 µM.

In one embodiment, the basal medium of the third culture medium comprises at least one of Iscove's Modified Dulbecco's Medium (IMDM), Eagle's Basal Medium (BME), MEM, DMEM, Ham's F-12 Medium, RPMI1640 Medium, Fischer's Medium, and DMEM/F12 Medium. In one preferred embodiment, the basal medium of the third culture medium comprises RPMI1640 medium.

In one embodiment, the basal medium of the third culture medium comprises at least one, at least two, at least three, or at least four of the following: a non-essential amino acid, glutamine, vitamin C, and a B27 medium supplement. In one preferred embodiment, the basal medium of the third culture medium comprises a non-essential amino acid, glutamine, vitamin C, and a B27 medium supplement.

In one embodiment, the B27 medium supplement is a vitamin A-free B27 medium supplement. In one embodiment, the concentration of the non-essential amino acid in the third culture medium is 0.2%-5% (v/v), preferably 0.5%-2% (v/v), and more preferably 1% (v/v).

In one embodiment, the concentration of glutamine in the third culture medium is 0.2%-5% (v/v), preferably 0.5%-2% (v/v), and more preferably 1% (v/v).

In one embodiment, the concentration of vitamin C in the third culture medium is 10-100 µg/mL, preferably 20-50 µg/mL, and more preferably 50 µg/mL.

In one embodiment, the concentration of the B27 medium supplement in the third culture medium is 1%-10% (v/v), preferably 2% (v/v).

In the present disclosure, the third culture medium is used for inducing the differentiation of a mesodermal cell into a hemogenic endothelial cell.

In one embodiment, the kit further comprises a first culture medium, wherein the first culture medium is a basal medium comprising a ROCK inhibitor.

In one embodiment, the ROCK inhibitor comprises at least one of blebbistatin, HA-100, Y-27632, HA-1077, KD-025, Y-33075, and narciclasine. In one preferred embodiment, the ROCK inhibitor comprises Y-27632.

In one embodiment, the concentration of the ROCK inhibitor in the first culture medium is 1-50 µM, preferably 5 µM-20 µM, and more preferably 10 µM.

In one embodiment, the basal medium of the first culture medium comprises at least one of E8 medium, mTESR medium, StemFit Basic 03, StemFit Basic 04, NutriStem hPSC XF medium, StemMACS iPS Brew medium, Stem-Partner ACF medium, TeSR-AOF medium, and TeSR2 medium. In one preferred embodiment, the basal medium of the first culture medium comprises StemFit Basic 03.

In the present disclosure, the first culture medium is used for acquiring an embryoid body.

In one embodiment, the stem cell described herein is a stem cell with multilineage differentiation potential.

In one embodiment, the stem cell with multilineage differentiation potential comprises at least one of an embryonic stem cell, a parthenogenetic stem cell, an induced pluripotent stem cell, a mesenchymal stem cell, an adipose-derived stem cell, and an umbilical cord blood stem cell. In one preferred embodiment, the stem cell with multilineage differentiation potential comprises an induced pluripotent stem cell.

In one embodiment, the stem cell is derived from a mammal, preferably from a primate, and more preferably from a human.

In one embodiment, the stem cell is a commercial human embryonic stem cell (hESC; e.g., H1 and H9) and/or a human induced pluripotent stem cell (hiPSC; e.g., WC50 and IMR90).

In one embodiment, the embryonic stem cell is a stem cell isolated or obtained from a human embryo within 14 days from fertilization without *in vivo* development.

In one specific embodiment, the kit comprises a first culture medium, a second culture medium, a third culture medium, a fourth culture medium, and a fifth culture medium; the first culture medium is a StemFit Basic 03 medium comprising 10 µM of Y27632 2HCl; the second culture medium is an RPMI1640 medium comprising 1% (v/v) of a non-essential amino acid, 1% (v/v) of glutamine, 50 µg/mL of vitamin C, 2% (v/v) of a vitamin A-free B27 medium supplement, 20 ng/mL of BMP4, and 3 µM of CHIR-99021; the third culture medium is an RPMI1640 medium comprising 1% (v/v) of a non-essential amino acid, 1% (v/v) of glutamine, 50 µg/mL of vitamin C, 2% (v/v) of a vitamin A-free B27 medium supplement, 5 ng/mL of BMP4, 50 ng/mL of VEGF-165, 50 ng/mL of bFGF, and 10 µM of SB431542; the fourth culture medium is an IMDM medium comprising 1% (v/v) of a non-essential amino acid, 1% (v/v) of glutamine, 50 µg/mL of vitamin C, 2% (v/v) of a vitamin A-free B27 medium supplement, 30 µM of N-acetyl-L-cysteine (NAC), 5 ng/mL of BMP4, 10 ng/mL of VEGF-165, 20 ng/mL of SCF, 5 ng/mL of IL3, 10 ng/mL of IL15, 20 ng/mL of IL7, and 10 ng/mL of FLT3L; the fifth culture medium is an IMDM medium comprising 1% (v/v) of a non-essential amino acid, 1% (v/v) of glutamine, 50 µg/mL of vitamin C, 2% (v/v) of a vitamin A-free B27 medium supplement, 30 µM of NAC, 5 ng/mL of BMP4, 10 ng/mL of VEGF, 20 ng/mL of SCF, 10 ng/mL of IL15, 20 ng/mL of IL7, 10 ng/mL of FLT3L, 1 µM of UM171, and 1 µM of SR1.

In one specific embodiment, the kit comprises a first culture medium, a second culture medium, a third culture medium, a fourth culture medium, and a fifth culture medium; the first culture medium is a StemFit Basic 03 medium comprising 10 µM of Y27632 or Y27632 ·2HCl; the second culture medium is an RPMI1640 medium comprising 1% of a non-essential amino acid, 1% of glutamine, 50 µg/mL of vitamin C, 2% (v/v) of a vitamin A-free B27 medium supplement, 20 ng/mL of BMP4, and 3 µM of CHIR-99021; the third culture medium is an RPMI1640 medium comprising 1% of a non-essential amino acid, 1% of glutamine, 50 µg/mL of vitamin C, 2% (v/v) of a vitamin A-free B27 medium supplement, 5 ng/mL of BMP4, 50 ng/mL of VEGF, 50 ng/mL of bFGF, and 10 µM of SB431542; the fourth culture medium is an IMDM medium comprising 1% of a non-essential amino acid, 1% of glutamine, 50 µg/mL of vitamin C, 2% (v/v) of a vitamin A-free B27 medium supplement, 5 ng/mL of BMP4, 10 ng/mL of VEGF, 20 ng/mL of SCF, 5 ng/mL of IL3, 10 ng/mL of IL15, 20 ng/mL of IL7, and 10 ng/mL of FLT3L; the fifth culture medium is an IMDM medium comprising 1% of a non-essential amino acid, 1% of glutamine, 50 µg/mL of vitamin C, 2% (v/v) of a vitamin A-free B27 medium supplement, 5 ng/mL of BMP4, 10 ng/mL of VEGF, 20 ng/mL of SCF, 10 ng/mL of IL15, 20 ng/mL of IL7, 10 ng/mL of FLT3L, 1 µM of UM171, 1 µM of SR1, and 30 µM of NAC.

In a second aspect, the present disclosure provides use of the kit of the present disclosure in preparing an embryoid body, preparing a mesodermal cell, preparing a hemogenic endothelial cell, preparing a natural killer (NK) cell, preparing a product for inducing the differentiation of a stem cell into an embryoid body, preparing a product for inducing the differentiation of a stem cell into a mesodermal cell, preparing a product for inducing the differentiation of a stem cell into a hemogenic endothelial cell, or preparing a product for inducing the differentiation of a stem cell into an NK cell.

In one embodiment, the stem cell described herein is a stem cell with multilineage differentiation potential.

In one embodiment, the stem cell with multilineage differentiation potential comprises at least one of an embryonic stem cell, a parthenogenetic stem cell, an induced pluripotent stem cell, a mesenchymal stem cell, an adipose-derived stem cell, and an umbilical cord blood stem cell. In one preferred embodiment, the stem cell with multilineage differentiation potential comprises an induced pluripotent stem cell.

In one embodiment, the stem cell is derived from a mammal, preferably from a primate, and more preferably from a human.

In one embodiment, the stem cell is a commercial human embryonic stem cell (hESC; e.g., H1 and H9) and/or a human induced pluripotent stem cell (hiPSC; e.g., WC50 and IMR90).

In one embodiment, the human embryonic stem cell is a stem cell isolated or obtained from a human embryo within 14 days from fertilization without *in vivo* development.

### Methods for inducing differentiation of stem cell into natural killer cell

In a third aspect, the present disclosure provides a method for inducing the differentiation of a stem cell into a natural killer cell, comprising: using the kit according to the first aspect of the present disclosure.

In one embodiment, the method comprises:
step (1), culturing a stem cell to give an embryoid body;
step (2), subjecting the embryoid body to mesoderm differentiation culture to give a mesodermal cell;
step (3), subjecting the mesodermal cell to hemogenic endothelium differentiation culture to give a hemogenic endothelial cell; and
step (4), culturing the hemogenic endothelial cell in the fourth culture medium and the fifth culture medium of the kit according to the first aspect of the present disclosure to give the NK cell.

In one embodiment, the culture in step (1) is performed in the first culture medium of the kit according to the first aspect of the present disclosure.

In one embodiment, the culture in step (2) is performed in the second culture medium of the kit according to the first aspect of the present disclosure.

In one embodiment, the culture in step (3) is performed in the third culture medium of the kit according to the first aspect of the present disclosure.

In one embodiment, the time for the culture in step (1) is 12-36 h, preferably 20-28 h, and more preferably 20-24 h.

In one embodiment, the time for the culture in step (2) is 24-72 h, preferably 36-60 h, and more preferably 42-48 h.

In one embodiment, the time for the culture in step (3) is 48-144 h, preferably 72-120 h, and more preferably 90-96 h.

In one embodiment, in step (4), culturing the hemogenic endothelial cell in the fourth culture medium and the fifth culture medium of the kit according to the first aspect of the present disclosure comprises sequentially culturing the hemogenic endothelial cell in the fourth culture medium and the fifth culture medium of the kit according to the first aspect of the present disclosure.

In one embodiment, the time for the culture of the hemogenic endothelial cell in the fourth culture medium is 96-192 h, preferably 120-192 h, and more preferably 144-168 h.

In one embodiment, the time for the culture of the hemogenic endothelial cell in the fifth culture medium is 144-432 h, preferably 216-360 h, and more preferably 312-336 h.

In one embodiment, during the culture of the hemogenic endothelial cell in the fifth culture medium, the medium is refreshed once every 5-7 days.

In one embodiment, the culture is performed in conditions of 35-38 °C and 4%-6% CO₂.

In one embodiment, the cultures in steps (1), (2), and (3) are suspension cultures.

In one embodiment, the cultures in steps (1), (2), and (3) are performed in a low-attachment plate.

In one embodiment, the culture in step (4) is an adherent culture.

In one embodiment, the culture in step (4) is performed in a normal-attachment plate.

In one embodiment, the stem cell described herein is a stem cell with multilineage differentiation potential.

In one embodiment, the stem cell with multilineage differentiation potential comprises at least one of an embryonic stem cell, a parthenogenetic stem cell, an induced pluripotent stem cell, a mesenchymal stem cell, an adipose-derived stem cell, and an umbilical cord blood stem cell. In one preferred embodiment, the stem cell with multilineage differentiation potential comprises an induced pluripotent stem cell.

In one embodiment, the stem cell is derived from a mammal, preferably from a primate, and more preferably from a human.

In one embodiment, the stem cell is a commercial human embryonic stem cell (hESC; e.g., H1 and H9) and/or a human induced pluripotent stem cell (hiPSC; e.g., WC50 and IMR90).

In one embodiment, the human embryonic stem cell is a stem cell isolated or obtained from a human embryo within 14 days from fertilization without *in vivo* development.

### NK cell

In a fourth aspect, the present disclosure provides a natural killer (NK) cell, prepared using the kit according to the first aspect of the present disclosure and/or the method according to the third aspect of the present disclosure.

In one embodiment, the content of CD45⁺/CD56⁺ cells in the NK cells is 80% or higher, 85% or higher, 90% or higher, 91% or higher, 92% or higher, 93% or higher, 94% or higher, 95% or higher, or 96% or higher, preferably 96% or higher, and more preferably 96.72%.

In one embodiment, the content of CD45⁺/CD3⁻ cells in the NK cells is 80% or higher, 85% or higher, 90% or higher, 91% or higher, 92% or higher, 93% or higher, 94% or higher, 95% or higher, 96% or higher, 97% or higher, or 98% or higher, preferably 98% or higher, and more preferably 98.87%.

In one embodiment, the content of CD56⁺/CD3⁻ cells in the NK cells is 80% or higher, 85% or higher, 90% or higher, 91% or higher, 92% or higher, 93% or higher, 94% or higher, 95% or higher, 96% or higher, or 97% or higher, preferably 97% or higher, and more preferably 97.12%. In one embodiment, the content of CD56⁺/CD94⁺ cells in the NK cells is 30% or higher, 35% or higher, 40% or higher, 45% or higher, 50% or higher, 51% or higher, 52% or higher, or 53% or higher, preferably 53% or higher, and more preferably 53.45%.

In one embodiment, the content of CD45⁺/CD94⁺ cells in the NK cells is 30% or higher, 35% or higher, 40% or higher, 45% or higher, 46% or higher, or 47% or higher, preferably 47% or higher, and more preferably 47.90%.

In one embodiment, the content of CD3⁻/CD16⁻ cells in the NK cells is 90% or higher, 95% or higher, or 96% or higher, preferably 96% or higher, and more preferably 96.47%.

In one embodiment, the content of CD3⁻/CD45⁺/CD56⁺ cells in the NK cells is 80% or higher, 85% or higher, or 90% or higher, preferably 90% or higher, and more preferably 90.64%.

In one embodiment, the content of CD3⁻/Nkp46⁺ cells in the NK cells is 60% or higher, 65% or higher, or 70% or higher, preferably 70% or higher, and more preferably 73.96%.

In one embodiment, the content of CD3⁻/CD56⁺/Nkp44⁺ cells in the NK cells is 80% or higher, 85% or higher, 90% or higher, 91% or higher, 92% or higher, 93% or higher, 94% or higher, 95% or higher, 96% or higher, or 97% or higher, preferably 93% or higher, and more preferably 93.35%.

In one embodiment, the content of CD3⁻/CD56⁺/Nkp30⁺ cells in the NK cells is 80% or higher, 85% or higher, 90% or higher, 91% or higher, 92% or higher, 93% or higher, 94% or higher, 95% or higher, 96% or higher, or 97% or higher, preferably 85% or higher, and more preferably 87.96%.

In one embodiment, the content of CD3⁻/CD56⁺/Nkp44⁺/Nkp30⁺ cells in the NK cells is 80% or higher, 85% or higher, 90% or higher, 91% or higher, 92% or higher, 93% or higher, 94% or higher, 95% or higher, 96% or higher, or 97% or higher, preferably 85% or higher, and more preferably 85.77%.

In one embodiment, the NK cell comprises an NK cell population.

### Pharmaceutical composition

In a fifth aspect, the present disclosure provides a pharmaceutical composition, comprising the NK cell according to the fourth aspect of the present disclosure.

In one embodiment, the pharmaceutical composition further comprises a pharmaceutically acceptable carrier.

In one embodiment, the pharmaceutical composition is a liquid formulation.

In one embodiment, the pharmaceutical composition is an agent for intravenous injection.

In one embodiment, the pharmaceutically acceptable carrier includes, but is not limited to: a saline, a buffer, glucose, water, DMSO, and a combination thereof.

In one embodiment, the concentration of the NK cells in the pharmaceutical composition is 1 × 10³ to 1 × 10⁷ cells/µL, preferably 1 × 10⁴ to 1 × 10⁶ cells/µL, and more preferably 1 × 10⁵ to 9.9 × 10⁵ cells/µL.

In one embodiment, the pharmaceutical composition further comprises an anti-tumor drug.

In one embodiment, the anti-tumor drug comprises at least one of camptothecin, temozolomide, a PARP inhibitor, an ATR inhibitor, a CDK4/6 inhibitor, cyclophosphamide, doxorubicin, cisplatin, and paclitaxel; preferably, the drug comprises temozolomide.

In one embodiment, the NK cell and the anti-tumor drug are present in a mixture or separately. In one embodiment, the tumor comprises at least one of a hematological tumor and a solid tumor; preferably, the tumor comprises at least one of non-small cell lung cancer, small cell lung cancer, colorectal cancer, melanoma, breast cancer, esophageal cancer, gastric cancer, bladder cancer, endometrial cancer, head and neck cancer, cervical cancer, liver cancer, renal cancer, and glioma; more preferably, the tumor comprises cervical cancer and glioma.

### Pharmaceutical combination

In a seventh aspect, the present disclosure provides a pharmaceutical combination for use in treating glioblastoma, wherein the pharmaceutical combination comprises an active ingredient; the active ingredient comprises a natural killer (NK) cell and a chemotherapeutic agent; the chemotherapeutic agent comprises temozolomide.

As used herein, the term "active ingredient" refers to any substance or mixture of substances in a pharmaceutical combination, which have pharmacological activity in the diagnosis, treatment, symptom alleviation, recurrence prevention, or prevention of a disease, and may function directly or indirectly.

In one embodiment, the glioblastoma is TMZ-resistant.

In one embodiment, the glioblastoma is TMZ-resistant, and TMZ induces the up-regulation of the expression of one or more of the immune ligands selected from CD112, CD155, CD54, CD70, B7-H6, CD48, PD-L1, and CD1d in the glioblastoma.

In one embodiment, the NK cell is an unmodified NK cell.

As used herein, the term "unmodified" means that no gene modification, which is achieved by intentionally incorporating exogenous genetic material in the form of, e.g., DNA or RNA, to the total genetic material of a cell to insert a gene or by editing (directionally engineering) a gene, is performed. For example, the term "unmodified" means that no gene modification that may lead to the insertion or deletion of a DNA fragment or the deletion, replacement, or the like, of a DNA base, is intentionally performed.

In one embodiment, the NK cell includes, but is not limited to, an NK-92 cell, a peripheral blood- or umbilical cord blood-derived NK cell, and an induced pluripotent stem cell (iPSC)- or embryonic stem cell-derived NK cell.

In one embodiment, the embryonic stem cell is a commercial human embryonic stem cell (hESC; e.g., H1 and H9).

In one embodiment, the embryonic stem cell is a stem cell isolated or obtained from a human embryo within 14 days from fertilization without *in vivo* development.

In one embodiment, the induced pluripotent stem cell (iPSC) is a human induced pluripotent stem cell (hiPSC; e.g., WC50 and IMR90).

In one embodiment, the NK cell is an induced pluripotent stem cell (iPSC)-derived NK cell. In one embodiment, the NK cell is an unmodified, induced pluripotent stem cell-derived NK cell.

In one embodiment, the induced pluripotent stem cell-derived NK cell is prepared using the kit according to the first aspect of the present disclosure or the method according to the third aspect of the present disclosure.

In one embodiment, the pharmaceutical combination further comprises an additional chemotherapeutic agent. The additional chemotherapeutic agent includes, but is not limited to, an anti-cancer agent, a metabolic antagonist, a DNA demethylating agent, a plant-derived anti-tumor agent, an alkylating agent, an antimetabolite, an anti-cancer antibiotic, a topoisomerase inhibitor, a mitotic inhibitor, a differentiation agent, and a hormonal therapeutic agent. Examples are: anti-tumor agents (e.g., actinomycin D, vincristine, vinblastine, alkylating agents, platinum-based compounds, antimetabolites, and nucleoside analogs, such as methotrexate and purine and pyrimidine analogs), antineoplastic agents (e.g., vincristine, doxorubicin, mitoxantrone, camptothecin, cisplatin, bleomycin, cyclophosphamide, methotrexate, and streptozotocin), antimetabolites (e.g., methotrexate, 6-mercaptopurine, 6-thioguanine, cytarabine, 5-fluorouracil, and dacarbazine), alkylating agents (e.g., mechlorethamine, thiotepa, chlorambucil, rachelmycin (CC-1065), melphalan, carmustine (BCNU), lomustine (CCNU), cyclophosphamide, busulfan, dibromomannitol, streptozotocin, mitomycin C, and cis-dichlorodiamine platinum(II) (DDP, cisplatin)), anthracyclines (e.g., daunorubicin (formerly known as daunomycin) and doxorubicin), antibiotics (e.g., dactinomycin (formerly actinomycin), bleomycin, mithramycin, and anthramycin (AMC)), and anti-mitosis agents (e.g., vincristine, vinblastine, paclitaxel, and maytansinoids). In one embodiment, the additional chemotherapeutic agent is selected from an anti-cancer agent, a metabolic antagonist, a DNA demethylating agent, a plant-derived anti-tumor agent, an alkylating agent, an antimetabolite, an anti-cancer antibiotic, a topoisomerase inhibitor, a mitotic inhibitor, a differentiation agent, and a hormonal therapeutic agent. These additional chemotherapeutic agents may further include, but are not limited to: carmustine, lomustine, vorinostat, mebendazole, imatinib mesylate, AG119, or any therapeutic derivatives thereof.

In one embodiment, the additional chemotherapeutic agent is selected from carmustine, lomustine, vorinostat, mebendazole, imatinib mesylate, AG119, and any therapeutic derivative thereof.

In one embodiment, the active ingredient does not comprise trimetrexate.

In one embodiment, the active ingredient does not comprise an immune checkpoint inhibitor. In one embodiment, the chemotherapeutic agent is temozolomide alone.

In one embodiment, the active ingredient consists of a natural killer cell and a chemotherapeutic agent.

In one embodiment, the active ingredient consists of a natural killer cell and temozolomide.

In one embodiment, the active ingredient is an NK-92 cell and temozolomide, a peripheral blood-derived or umbilical cord blood-derived NK cell and temozolomide, an induced pluripotent stem cell (iPSC)-derived NK cell and temozolomide, or an embryonic stem cell-derived NK cell and temozolomide.

In one embodiment, the active ingredient consists of an induced pluripotent stem cell-derived NK cell and temozolomide.

In one embodiment, the active ingredient consists of an unmodified, induced pluripotent stem cell-derived NK cell and temozolomide.

In one embodiment, the active ingredient consists of an induced pluripotent stem cell-derived NK cell prepared using the kit according to the first aspect of the present disclosure and temozolomide.

In one embodiment, the active ingredient consists of an induced pluripotent stem cell-derived NK cell prepared using the method for inducing the differentiation of a stem cell into a natural killer cell according to the third aspect of the present disclosure and temozolomide.

In one embodiment, the active ingredient consists of an unmodified, induced pluripotent stem cell-derived NK cell prepared using the kit according to the first aspect of the present disclosure and temozolomide.

In one embodiment, the active ingredient consists of an unmodified, induced pluripotent stem cell-derived NK cell prepared using the method for inducing the differentiation of a stem cell into a natural killer cell according to the third aspect of the present disclosure and temozolomide.

In one embodiment, the pharmaceutical combination is for use in treating glioblastoma; the glioblastoma is TMZ-resistant; the pharmaceutical combination comprises an active ingredient consisting of an induced pluripotent stem cell-derived NK cell and temozolomide.

In one embodiment, the pharmaceutical combination is for use in treating glioblastoma; the glioblastoma is TMZ-resistant; the pharmaceutical combination comprises an active ingredient consisting of an unmodified, induced pluripotent stem cell-derived NK cell and temozolomide. In one embodiment, the active ingredients in the pharmaceutical combination can be used simultaneously or separately (e.g., sequentially).

In one embodiment, the pharmaceutical combination is a kit.

In a tenth aspect, the present disclosure provides a kit, comprising the natural killer cell and the chemotherapeutic agent as defined in the seventh aspect of the present disclosure or the pharmaceutical combination according to the seventh aspect, and a package insert. The kit may further comprise a suitable container. In some certain embodiments, the kit further comprises a device for administration. The kit generally comprises a label indicative of the intended use and/or method of use of the kit contents. The term "label" includes any written or recorded materials provided on or with the kit or otherwise provided with the kit.

### Methods for Treating Glioblastoma

In an eleventh aspect, the present disclosure further provides a method for treating glioblastoma in a subject in need, comprising:
administering to the subject a natural killer (NK) cell; and
administering to the subject a chemotherapeutic agent treatment,
wherein the chemotherapeutic agent comprises temozolomide (TMZ).

In one embodiment, the chemotherapeutic agent treatment is administered before the administration of the NK cell to the subject, after the administration of the NK cell to the subject, or concurrently with the administration of the NK cell to the subject, or any combination of the aforementioned modes.

In one embodiment, the glioblastoma is TMZ-resistant.

In one embodiment, the glioblastoma is TMZ-resistant, and TMZ induces the up-regulation of the expression of one or more of the immune ligands selected from CD112, CD155, CD54, CD70, B7-H6, CD48, PD-L1, and CD1d in the glioblastoma.

In one embodiment, the NK cell is an unmodified NK cell.

In one embodiment, the NK cell includes, but is not limited to, an NK-92 cell, a peripheral blood-derived or umbilical cord blood-derived NK cell, and an induced pluripotent stem cell (iPSC)- or embryonic stem cell-derived NK cell.

In one embodiment, the embryonic stem cell is a commercial human embryonic stem cell (hESC; e.g., H1 and H9).

In one embodiment, the embryonic stem cell is a stem cell isolated or obtained from a human embryo within 14 days from fertilization without *in vivo* development.

In one embodiment, the induced pluripotent stem cell (iPSC) is a human induced pluripotent stem cell (hiPSC; e.g., WC50 and IMR90).

In one embodiment, the NK cell is an induced pluripotent stem cell (iPSC)-derived NK cell. In one embodiment, the NK cell is an unmodified, induced pluripotent stem cell-derived NK cell.

In one embodiment, the induced pluripotent stem cell-derived NK cell is prepared using the kit according to the first aspect of the present disclosure.

In one embodiment, the induced pluripotent stem cell-derived NK cell is prepared using the method for inducing the differentiation of a stem cell into a natural killer cell according to the third aspect of the present disclosure.

In one embodiment, the method further comprises administering to the subject an additional chemotherapeutic agent treatment. The additional chemotherapeutic agent includes, but is not limited to, an anti-cancer agent, a metabolic antagonist, a DNA demethylating agent, a plant-derived anti-tumor agent, an alkylating agent, an antimetabolite, an anti-cancer antibiotic, a topoisomerase inhibitor, a mitotic inhibitor, a differentiation agent, and a hormonal therapeutic agent. Examples are: anti-tumor agents (e.g., actinomycin D, vincristine, vinblastine, alkylating agents, platinum-based compounds, antimetabolites, and nucleoside analogs, such as methotrexate and purine and pyrimidine analogs), antineoplastic agents (e.g., vincristine, doxorubicin, mitoxantrone, camptothecin, cisplatin, bleomycin, cyclophosphamide, methotrexate, and streptozotocin), antimetabolites (e.g., methotrexate, 6-mercaptopurine, 6-thioguanine, cytarabine, 5-fluorouracil, and dacarbazine), alkylating agents (e.g., mechlorethamine, thiotepa, chlorambucil, rachelmycin (CC-1065), melphalan, carmustine (BCNU), lomustine (CCNU), cyclophosphamide, busulfan, dibromomannitol, streptozotocin, mitomycin C, and cis-dichlorodiamine platinum(II) (DDP, cisplatin)), anthracyclines (e.g., daunorubicin (formerly known as daunomycin) and doxorubicin), antibiotics (e.g., dactinomycin (formerly actinomycin), bleomycin, mithramycin, and anthramycin (AMC)), and anti-mitosis agents (e.g., vincristine, vinblastine, paclitaxel, and maytansinoids). In one embodiment, the additional chemotherapeutic agent is selected from an anti-cancer agent, a metabolic antagonist, a DNA demethylating agent, a plant-derived anti-tumor agent, an alkylating agent, an antimetabolite, an anti-cancer antibiotic, a topoisomerase inhibitor, a mitotic inhibitor, a differentiation agent, and a hormonal therapeutic agent. These additional chemotherapeutic agents may further include, but are not limited to: carmustine, lomustine, vorinostat, mebendazole, imatinib mesylate, AG119, or any therapeutic derivatives thereof.

In one embodiment, the additional chemotherapeutic agent is selected from carmustine, lomustine, vorinostat, mebendazole, imatinib mesylate, AG119, and any therapeutic derivative thereof.

In one embodiment, the additional chemotherapeutic agent treatment is administered before the administration of the NK cell or temozolomide (TMZ) to the subject, after the administration of the NK cell or temozolomide (TMZ) to the subject, or concurrently with the administration of the NK cell or temozolomide (TMZ) to the subject, or any combination of the aforementioned modes.

In one embodiment, the method does not comprise administering to the subject trimetrexate. In one embodiment, the method does not comprise administering to the subject an immune checkpoint inhibitor.

In one embodiment, the chemotherapeutic agent is temozolomide (TMZ).

In one embodiment, the method further comprises:
administering to the subject a natural killer (NK) cell; and
administering to the subject a chemotherapeutic agent treatment, wherein the chemotherapeutic agent treatment is administered before the administration of the NK cell to the subject, after the administration of the NK cell to the subject, or concurrently with the administration of the NK cell to the subject, or any combination of the aforementioned modes; the chemotherapeutic agent is temozolomide.

In one embodiment, the method further comprises:
administering to the subject an induced pluripotent stem cell-derived NK cell; and
administering to the subject a chemotherapeutic agent treatment, wherein the chemotherapeutic agent treatment is administered before the administration of the induced pluripotent stem cell-derived NK cell to the subject, after the administration of the induced pluripotent stem cell-derived NK cell to the subject, or concurrently with the administration of the induced pluripotent stem cell-derived NK cell to the subject, or any combination of the aforementioned modes; the chemotherapeutic agent is temozolomide.

In one embodiment, the method for treating glioblastoma in the subject in need, comprises:
administering to the subject an induced pluripotent stem cell-derived NK cell; and
administering to the subject a chemotherapeutic agent treatment, wherein the chemotherapeutic agent treatment is administered before the administration of the induced pluripotent stem cell-derived NK cell to the subject, after the administration of the induced pluripotent stem cell-derived NK cell to the subject, or concurrently with the administration of the induced pluripotent stem cell-derived NK cell to the subject, or any combination of the aforementioned modes;
the chemotherapeutic agent is temozolomide (TMZ);
the glioblastoma is TMZ-resistant.

In one embodiment, the method for treating glioblastoma in the subject in need, comprises:
administering to the subject an unmodified, induced pluripotent stem cell-derived NK cell; and
administering to the subject a chemotherapeutic agent treatment, wherein the chemotherapeutic agent treatment is administered before the administration of the unmodified, induced pluripotent stem cell-derived NK cell to the subject, after the administration of the unmodified, induced pluripotent stem cell-derived NK cell to the subject, or concurrently with the administration of the unmodified, induced pluripotent stem cell-derived NK cell to the subject, or any combination of the aforementioned modes;
the chemotherapeutic agent is temozolomide (TMZ);
the glioblastoma is TMZ-resistant.

In one embodiment, the method for treating glioblastoma in the subject in need, comprises:
administering to the subject an induced pluripotent stem cell-derived NK cell; and
administering to the subject a chemotherapeutic agent treatment, wherein the chemotherapeutic agent treatment is administered before the administration of the induced pluripotent stem cell-derived NK cell to the subject, after the administration of the induced pluripotent stem cell-derived NK cell to the subject, or concurrently with the administration of the induced pluripotent stem cell-derived NK cell to the subject, or any combination of the aforementioned modes;
the chemotherapeutic agent is temozolomide (TMZ);
the glioblastoma is TMZ-resistant;
the induced pluripotent stem cell-derived NK cell is prepared using the kit according to the first aspect of the present disclosure.

In one embodiment, the method for treating glioblastoma in the subject in need, comprises:
administering to the subject an induced pluripotent stem cell-derived NK cell; and
administering to the subject a chemotherapeutic agent treatment, wherein the chemotherapeutic agent treatment is administered before the administration of the induced pluripotent stem cell-derived NK cell to the subject, after the administration of the induced pluripotent stem cell-derived NK cell to the subject, or concurrently with the administration of the induced pluripotent stem cell-derived NK cell to the subject, or any combination of the aforementioned modes;
the chemotherapeutic agent is temozolomide (TMZ);
the glioblastoma is TMZ-resistant;
the induced pluripotent stem cell-derived NK cell is prepared using the method for inducing the differentiation of a stem cell into a natural killer cell according to the third aspect of the present disclosure.

In one embodiment, the chemotherapeutic agent treatment is administered 8 to 72 hours before the administration of the NK cell to the subject, 8 to 72 hours after the administration of the NK cell to the subject, or 8 to 72 hours from the administration of the NK cell to the subject.

In one embodiment, the chemotherapeutic agent treatment is administered 8, 16, 24, 32, 40, 48, 56, 64, or 72 hours before the administration of the NK cell to the subject, 8, 16, 24, 32, 40, 48, 56, 64, or 72 hours after the administration of the NK cell to the subject, or 8, 16, 24, 32, 40, 48, 56, 64, or 72 hours from the administration of the NK cell to the subject.

In one embodiment, temozolomide treatment is administered 8, 16, 24, 32, 40, 48, 56, 64, or 72 hours before the administration of the NK cell to the subject, 8, 16, 24, 32, 40, 48, 56, 64, or 72 hours after the administration of the NK cell to the subject, or 8, 16, 24, 32, 40, 48, 56, 64, or 72 hours from the administration of the NK cell to the subject.

In one embodiment, temozolomide treatment is administered 8, 16, 24, 32, 40, 48, 56, 64, or 72 hours before the administration of the induced pluripotent stem cell-derived NK cell to the subject, 8, 16, 24, 32, 40, 48, 56, 64, or 72 hours after the administration of the induced pluripotent stem cell-derived NK cell to the subject, or 8, 16, 24, 32, 40, 48, 56, 64, or 72 hours from the administration of the induced pluripotent stem cell-derived NK cell to the subject.

### Use and Treatment

In a sixth aspect, the present disclosure provides use of the NK cell according to the fourth aspect of the present disclosure or the pharmaceutical composition according to the fifth aspect of the present disclosure in preparing a medicament for preventing and/or treating a tumor.

In a ninth aspect, the present disclosure provides a method for treating a tumor, comprising: administering to a subject the NK cell according to the fourth aspect of the present disclosure or the pharmaceutical composition according to the fifth aspect of the present disclosure.

In one embodiment, the subject is a mammal, preferably a primate, and more preferably a human.

In one embodiment, the site of administration is a vein of the subject.

In one embodiment, the tumor comprises at least one of a hematological tumor and a solid tumor; preferably, the tumor comprises at least one of non-small cell lung cancer, small cell lung cancer, colorectal cancer, melanoma, breast cancer, esophageal cancer, gastric cancer, bladder cancer, endometrial cancer, head and neck cancer, cervical cancer, liver cancer, renal cancer, and glioma; more preferably, the tumor comprises cervical cancer and glioma.

In an eighth aspect, the present disclosure provides use of the natural killer (NK) cell and the chemotherapeutic agent as defined in the seventh aspect of the present disclosure, the pharmaceutical combination according to the seventh aspect, or the kit according to the tenth aspect in preparing a medicament for treating glioblastoma.

In one embodiment, the glioblastoma is TMZ-resistant.

In one embodiment, the glioblastoma is TMZ-resistant, and TMZ induces the up-regulation of the expression of one or more of the immune ligands selected from CD112, CD155, CD54, CD70, B7-H6, CD48, PD-L1, and CD1d in the glioblastoma.

In one embodiment, the chemotherapeutic agent is administered before, after, or concurrent with the administration of the NK cell.

In one embodiment, the chemotherapeutic agent is administered 8 to 72 h before, 8 to 72 h after, or 8 to 72 h from the administration of the NK cell.

In a twelfth aspect, the present disclosure provides the natural killer (NK) cell and the chemotherapeutic agent as defined in the seventh aspect of the present disclosure, the pharmaceutical combination according to the seventh aspect of the present disclosure, or the kit according to the tenth aspect of the present disclosure for use in treating glioblastoma.

In one embodiment, the glioblastoma is TMZ-resistant.

In one embodiment, the glioblastoma is TMZ-resistant, and TMZ induces the up-regulation of the expression of one or more of the immune ligands selected from CD112, CD155, CD54, CD70, B7-H6, CD48, PD-L1, and CD1d in the glioblastoma.

In one embodiment, the method for treating glioblastoma in the subject in need of the present disclosure, comprises:
administering to the subject a therapeutically effective amount of the NK cell as defined in the seventh aspect of the present disclosure; and
administering to the subject a therapeutically effective amount of the chemotherapeutic agent treatment as defined in the seventh aspect of the present disclosure, wherein the chemotherapeutic agent treatment is administered before the administration of the NK cell to the subject, after the administration of the NK cell to the subject, or concurrently with the administration of the NK cell to the subject, or any combination of the aforementioned modes.

As used herein, the term "treat", "treating", or "treatment" refers to ameliorating or reversing at least one measurable physical parameter associated with a cancer (e.g., glioblastoma or TMZ-resistant glioblastoma), which may be, but is unnecessarily, discernible in the subject. The term "treat", "treating", or "treatment" may also refer to causing the regression or preventing or at least slowing the progression of a disease, disorder, or condition. In one specific embodiment, the "treat", "treating", or "treatment" refers to alleviating, preventing the progression or development of, or reducing the duration of one or more symptoms associated with a disease, disorder, or condition (e.g., a tumor or more preferably, a cancer, e.g., glioblastoma or TMZ-resistant glioblastoma). In one specific embodiment, the "treat", "treating", or "treatment" refers to preventing the recurrence of a disease, disorder, or condition. In one specific embodiment, the "treat", "treating", or "treatment" refers to increasing the survival of a subject with the disease, disorder, or condition. In one specific embodiment, the "treat", "treating", or "treatment" refers to eliminating a disease, disorder, or condition in a subject.

The term "therapeutically effective amount" refers to an amount of an agent (e.g., a drug, a pharmaceutical combination, or an agent in a kit) sufficient to prevent or inhibit the development of a disease or symptom and/or slow, alleviate, or delay the progression or severity of the disease or symptom. The therapeutically effective amount is affected by factors including, but not limited to, the progression and severity of the disease or symptom, the age, sex, body weight and physiological condition of the subject, the duration of the treatment, and the specific route of administration. The therapeutically effective amount may be administered in one or more doses. The therapeutically effective amount may be achieved by continuous or intermittent doses.

According to particular embodiments, the therapeutically effective amount may refer to a therapeutic amount sufficient to achieve one, two, three, four, or more of the following effects: (i) reducing or ameliorating the severity of a disease, disorder, or condition of interest or an associated symptom; (ii) reducing the duration of a disease, disorder, or condition of interest or an associated symptom; (iii) preventing the progression of a disease, disorder, or condition of interest or an associated symptom; (iv) causing the regression of a disease, disorder, or condition of interest or an associated symptom; (v) preventing the progression or attack of a disease, disorder, or condition of interest or an associated symptom; (vi) preventing the recurrence of a disease, disorder, or condition of interest or an associated symptom; (vii) reducing the hospitalization of a subject suffering from a disease, disorder, or condition of interest or an associated symptom; (viii) reducing the duration of hospitalization of a subject suffering from a disease, disorder, or condition of interest or an associated symptom; (ix) increasing the survival of a subject suffering from a disease, disorder, or condition of interest or an associated symptom; (x) inhibiting or reducing a disease, disorder, or condition of interest or an associated symptom in a subject; and/or (xi) enhancing or improving the prophylactic or therapeutic effect of another therapy.

In one embodiment, the natural killer (NK) cells and the chemotherapeutic agent as defined in the seventh aspect of the present disclosure, the pharmaceutical combination according to the seventh aspect of the present disclosure, the method according to the eleventh aspect of the present disclosure, the kit according to the tenth aspect of the present disclosure, or the medicament as defined in the eighth aspect of the present disclosure may also be used for treating sensitive GBM that gradually develops drug resistance and various strategies to escape from the effect of TMZ and finally transforms into resistant GBM.

The NK cell of the present application and/or the pharmaceutical combination of the present application may be administered in any physiologically acceptable carrier. The cell population comprising the NK cell of the present application may comprise a purified cell population. Those skilled in the art can readily determine the cell in a cell population using a variety of well known methods, which may have a purity range of about 50% to 55%, about 55% to 60%, about 60% to 65%, about 65% to 70%, about 70% to 75%, about 75% to 80%, about 80% to 85%, about 85% to 90%, about 90% to 95%, or about 95% to 100%. The dose can be readily adjusted by those skilled in the art. For example, a decrease in purity may require an increase in dose. In the present application, the NK cell and/or the pharmaceutical combination comprising the NK cell may be administered to the subject at a dose in cells/kg body weight of the subject. Generally, the cell dose is in the range of about 10⁴ to about 10¹⁰ cells/kg body weight, e.g., about 10⁵ to about 10⁹, about 10⁵ to about 10⁸, about 10⁵ to about 10⁷, or about 10⁵ to about 10⁶, depending on the route and site of administration. Exemplary dose ranges include, but are not limited to, 1 × 10⁴ to 1 × 10⁸, 2 × 10⁴ to 1 × 10⁸, 3 × 10⁴ to 1 × 10⁸, 4 × 10⁴ to 1 × 10⁸, 5 × 10⁴ to 6 × 10⁸, 7 × 10⁴ to 1 × 10⁸, 8 × 10⁴ to 1 × 10⁸, 9 × 10⁴ to 1 × 10⁸, 1 × 10⁵ to 1 × 10⁸, 1 × 10⁵ to 9 × 10⁷, 1 × 10⁵ to 8 × 10⁷, 1 × 10⁵ to 7 × 10⁷, 1 × 10⁵ to 6 × 10⁷, 1 × 10⁵ to 5 × 10⁷, 1 × 10⁵ to 4 × 10⁷, 1 × 10⁵ to 4 × 10⁷, 1 × 10⁵ to 3 × 10⁷, 1 × 10⁵ to 2 × 10⁷, 1 × 10⁵ to 1 × 10⁷, 1 × 10⁵ to 9 × 10⁶, 1 × 10⁵ to 8 × 10⁶, 1 × 10⁵ to 7 × 10⁶, 1 × 10⁵ to 6 × 10⁶, 1 × 10⁵ to 5 × 10⁶, 1 × 10⁵ to 4 × 10⁶, 1 × 10⁵ to 4 × 10⁶, 1 × 10⁵ to 3 × 10⁶, 1 × 10⁵ to 2 × 10⁶, 1 × 10⁵ to 1 × 10⁶, 2 × 10⁵ to 9 × 10⁷, 2 × 10⁵ to 8 × 10⁷, 2 × 10⁵ to 7 × 10⁷, 2 × 10⁵ to 6 × 10⁷, 2 × 10⁵ to 5 × 10⁷, 2 × 10⁵ to 4 × 10⁷, 2 × 10⁵ to 4 × 10⁷, 2 × 10⁵ to 3 × 10⁷, 2 × 10⁵ to 2 × 10⁷, 2 × 10⁵ to 1 × 10⁷, 2 × 10⁵ to 9 × 10⁶, 2 × 10⁵ to 8 × 10⁶, 2 × 10⁵ to 7 × 10⁶, 2 × 10⁵ to 6 × 10⁶, 2 × 10⁵ to 5 × 10⁶, 2 × 10⁵ to 4 × 10⁶, 2 × 10⁵ to 4 × 10⁶, 2 × 10⁵ to 3 × 10⁶, 2 × 10⁵ to 2 × 10⁶, 2 × 10⁵ to 1 × 10⁶, 3 × 10⁵ to 3 × 10⁶ cells/kg, and the like. Furthermore, the dose can be adjusted with concerns about administration in a single dose or multiple doses. The effective dose may also be accurately determined based on individual factors of each subject.

As used herein, the term "effector-to-target ratio (E:T)" refers to the ratio of the effector cell and the target cell. In some embodiments, the effector-to-target ratio refers to the ratio of the NK cell used to the glioblastoma cell. Exemplary effector-to-target ratio ranges include, but are not limited to, 100:1, 90:1, 85:1, 80:1, 75:1, 70:1, 65:1, 60:1, 55:1, 50:1, 45:1, 40:1, 35:1, 30:1, 25:1, 20:1, 19:1, 18:1, 17:1, 16:1, 15:1, 14:1, 13:1, 12:1, 11:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:11, 1:12, 1:14, 1:15, 1:16, 1:17, 1:18, 1:19, and 1:20. In some embodiments, the effector-to-target ratio is 1:1, 1:4, or 1:8. In one embodiment, the effector-to-target ratio is 1:1.

In the present application, TMZ and/or the pharmaceutical combination comprising TMZ may be administered to the subject at a dose in mg/kg body weight of the subject. Exemplary doses include, but are not limited to, about 0.01, about 0.05, about 0.1, about 0.2, about 0.3, about 0.4, about 0.5, about 0.6, about 0.8, about 1, about 2, about 5, about 10, about 15, about 20, about 25, about 30, about 35, about 40, about 45, about 50, about 55, about 60, about 65, about 70, about 75, about 80, about 85, about 90, about 95, about 100, about 150, about 200, about 250, about 300, about 350, about 400, about 450, about 500, about 550, about 600, about 650, about 700, about 750, about 800, about 850, about 900, or about 1000 mg/kg of the subject. In the present application, TMZ and/or the pharmaceutical combination comprising TMZ may be administered to the subject at a dose in mg/m² body surface area of the subject. Exemplary doses include, but are not limited to, about 0.01, about 0.05, about 0.1, about 0.2, about 0.3, about 0.4, about 0.5, about 0.6, about 0.8, about 1, about 2, about 5, about 10, about 15, about 20, about 25, about 30, about 40, about 45, about 50, about 55, about 60, about 65, about 70, about 75, about 80, about 85, about 90, about 95, about 100, about 150, about 200, about 250, about 300, about 350, about 400, about 450, about 500, about 550, about 600, about 650, about 700, about 750, about 800, about 850, about 900, or about 1000 mg/m² of the subject.

In the present application, the amount of TMZ may be, for example, 0.001-5000 µM, such as 10-1000 µM, e.g., 10, 20, 30, 40, 50, 60, 70, 80, 90, 100, 150, 200, 250, 300, 350, 400, 450, 500, 650, 700, 750, 800, 850, 900, 950, or 1000 µM.

### Beneficial Effects

The present disclosure provides a kit for inducing the differentiation of a stem cell into a natural killer cell. The kit employs culture media with well-defined chemical compositions, small chemical molecules, and cytokines, without involving serum or other substances that are prone to cause instability such as inter-batch variations. In addition, the kit features cost-efficiency and reduced differentiation period compared to known commercial differentiation media.

The present disclosure provides an efficient and clear NK cell differentiation strategy based on the aforementioned kit. An embryoid body is obtained by culturing a stem cell. Then the embryoid body is subjected to mesoderm differentiation culture to give a mesodermal cell. The mesodermal cell is then subjected to hemogenic endothelium differentiation culture to give a hemogenic endothelial cell. Finally, the hemogenic endothelial cell is subjected to NK cell differentiation to give an NK cell. Due to the use of culture media with well-defined chemical compositions, small chemical molecules, and cytokines, the whole differentiation process does not involve the influence of serum (human AB serum), stromal proteins, and stromal cells, which makes the method for inducing differentiation more standardized and provides homogeneous functional NK cells of a large quantity for clinical therapeutic transformation. Also, the EB differentiation method is used in the hemogenic endothelial cell differentiation stage, so as to reduce the early differentiation period and increase the efficiency for acquiring hemogenic endothelial cells, thereby improving the differentiation quantity, differentiation quality, and differentiation purity of the subsequent NK cells. The contents of CD45⁺/CD94⁺ cells and CD56⁺/CD94⁺ cells in the NK cells acquired by the method are significantly increased, thus providing the NK cells with potent IFN-γ secretion functionality and better tumor cell-killing functionality. In addition, the acquired NK cells possess improved targeting and killing effects against tumor cells in combination with existing CAR therapies. Even more surprisingly, compared with the starting iPSC cells, the finally prepared mature NK cells of the present disclosure exhibited an expansion fold of more than 500, while requiring no conventional NK cell expansion steps, thereby greatly facilitating the cell transplantation and the treatment of related diseases. Meanwhile, the method for induction is also applicable to the current universal iPSCs, thereby inducing the differentiation into universal NK cells suitable for a variety of patients.

The NK cell of the present disclosure in combination with TMZ and the corresponding pharmaceutical combination or kit thereof can exhibit excellent effects, for example, but not limited to: (1) higher degranulation activity of NK cells; (2) higher tumor cell-killing activity of NK cells against resistant GBM; (3) resistance against the immunosuppressive effect of resistant GBM; (4) prolonged survival. In particular, the combination of the induced pluripotent stem cell-derived NK cell with TMZ has the most excellent tumor cell-killing activity against resistant GBM.

### Examples

The present disclosure is further illustrated with reference to the following examples. It will be appreciated that these examples are merely exemplary and are not intended to limit the present disclosure. Unless otherwise specified, the following materials and devices are commercially available or prepared according to methods well known in the art. Also, unless otherwise specified, the following experiments were conducted according to the manufacturer's instructions or according to methods and procedures well known in the art.

The human induced pluripotent stem cells (iPSCs) used in the examples were reprogrammed iPSCs by the company, and were prepared using the CTS^{™} CytoTune^{™}-iPS 2.1 Sendai reprogramming kit (Cat. No. A34546) (see the kit instructions for details). The starting cells were human peripheral blood cells.

In the examples, the low-attachment 96-well plates were purchased from Corning; BMP4 was purchased from StemImmune LLC., Cat# HST-B4-0100; CHIR-99021 was purchased from Selleck; non-essential amino acids were purchased from Gibco, Cat# 11140050; glutamine was purchased from Gibco; vitamin C was purchased from Sigma; the vitamin A-free B27 medium supplement was purchased from Gibco.

### Example 1. Kit for inducing differentiation of human induced pluripotent stem cells into natural killer cells

In this example, provided is a kit for inducing the differentiation of human induced pluripotent stem cells into natural killer cells, comprising: a first culture medium, a second culture medium, a third culture medium, a fourth culture medium, and a fifth culture medium.

The first culture medium was StemFit Basic 03 medium containing 10 µM of Y27632·2HCl (Ajinomoto, Cat. No. AK03N).

The second culture medium was a differentiation medium containing 20 ng/mL of BMP4 (bone morphogenetic protein 4) and 3 µM of CHIR-99021; the differentiation medium was an RPMI1640 medium containing 1% (v/v) of non-essential amino acids, 1% (v/v) of glutamine, 50 µg/mL of vitamin C, and 2% (v/v) of vitamin A-free B27 medium supplement.

The third culture medium was a differentiation medium containing 5 ng/mL of BMP4, 50 ng/mL of VEGF-165 (vascular endothelial growth factor), 50 ng/mL of bFGF (basic fibroblast growth factor), and 10 µM of SB431542; the differentiation medium was an RPMI1640 medium containing 1% (v/v) of non-essential amino acids, 1% (v/v) of glutamine, 50 µg/mL of vitamin C, and 2% (v/v) of vitamin A-free B27 medium supplement.

The fourth culture medium was a differentiation medium containing 5 ng/mL of BMP4, 10 ng/mL of VEGF-165, 20 ng/mL of SCF (stem cell factor), 5 ng/mL of IL3 (multi-colony-stimulating factor, multi-CSF), 10 ng/mL of IL15 (interleukin 15), 20 ng/mL of IL7 (interleukin 7), and 10 ng/mL of FLT3L (Fms-related tyrosine kinase 3 ligand); the differentiation medium was an IMDM (Iscove's modified Dulbecco's medium) medium containing 1% (v/v) of non-essential amino acids, 1% (v/v) of glutamine, 50 µg/mL of vitamin C, 2% (v/v) of vitamin A-free B27 medium supplement, and 30 µM of N-acetyl-L-cysteine.

The fifth culture medium was a differentiation medium containing 5 ng/mL of BMP4, 10 ng/mL of VEGF-165, 20 ng/mL of SCF, 10 ng/mL of IL15, 20 ng/mL of IL7, 10 ng/mL of FLT3L, 1 µM of UM171 (CAS No. 1448724-09-1), and 1 µM of SR1 (CAS No. 1227633-49-9); the differentiation medium was an IMDM medium containing 1% (v/v) of non-essential amino acids, 1% (v/v) of glutamine, 50 µg/mL of vitamin C, 2% (v/v) of vitamin A-free B27 medium supplement, and 30 µM of N-acetyl-L-cysteine.

### Example 2. Method for inducing differentiation of human induced pluripotent stem cells into natural killer cells

In this example, provided is a method for inducing the differentiation of human induced pluripotent stem cells into natural killer cells, comprising procedures of using the kit of Example 1, or specifically, the following steps (the schematic of the differentiation is shown in panel A of FIG. 1):
step (1), human induced pluripotent stem cells were in a low-attachment 96-well plate to give embryoid bodies:
   iPSCs were seeded into a low-attachment six-well plate at 2 × 10⁵ cells/well using the first culture medium and cultured at 37 °C and 5% CO₂ for one day to give embryoid bodies (D0).
   step (2), the embryoid bodies were subjected to mesoderm differentiation culture to give mesodermal cells:
      After one day, the first culture medium was replaced with the second culture medium, and the cells were cultured at 37 °C and 5% CO₂ for two days to give mesodermal cells (ME cells) (D2).
   step (3), the mesodermal cells were subjected to hemogenic endothelium differentiation culture to give hemogenic endothelial cells:
      After two days, the second culture medium was replaced with the third culture medium, and the cells were cultured at 37 °C and 5% CO₂ for four days to give hemogenic endothelial cells (HE cells).
   step (4), the hemogenic endothelial cells were subjected to NK cell differentiation culture to give NK cells:
      After four days, the embryoid bodies (the hemogenic endothelial cells obtained after four days of culture in step (3)) in the low-attachment 96-well plates were collected and added to a 50 mL centrifuge tube, and the cells were allowed to settle naturally for 1-2 min. Then the supernatant was discarded by pipetting, with about 5 mL of the supernatant remaining in the centrifuge tube. 14 mL of the fourth culture medium was then added, and the resulting cell pellet was mixed well by gently pipetting, evenly distributed into three normal-attachment 6-well plates, and cultured in an incubator at 37 °C and 5% CO₂ for 7 days. The cell morphology after 2 days of culture (iNK-D2, EB adherent D2) is shown in panel B in FIG. 1.
      After seven days, about 1 mL of supernatant was discarded by gently pipetting from the six-well plate, and the fifth culture medium was added to the six-well plate at 3 mL/well. The plate was placed back into the incubator and incubated at 37 °C and 5% CO₂ for 7 days.
      After seven days, the fifth culture medium was added to the six-well plate at 2 mL/well, and the plate was placed back into the incubator and incubated at 37 °C with 5% CO₂ for 7 days.
      The cell morphology after 6 days of culture (iNK-D20) is shown in panel C in FIG. 1.
   step (5), the differentiated NK cells were collected:
      On day 13, day 16, and day 20 of the NK cell differentiation culture for hemogenic endothelial cells, the supernatants (suspended cells) in the six-well plates were collected and added to centrifuge tubes. The tubes were centrifuged in a centrifuge at 1500 rpm for 10 min, and resuspended in 5 mL of the fifth culture medium. The cell suspensions were then filtered through a 70-µm cell strainer. The filtered cell suspensions were centrifuged in a centrifuge again at 1500 rpm for 5 min, and resuspended in 5 mL of the fifth culture medium. The cells were counted by AO/PI staining, and the cell viability and cell count were recorded. The subsequent cell assays, cell cryopreservation studies, and the like were performed as required.

### Example 3-1. Kit for inducing differentiation of human induced pluripotent stem cells into natural killer cells

In this example, provided is a kit for inducing the differentiation of human induced pluripotent stem cells into natural killer cells, similar to Example 1 but different in that the concentration of BMP4 (bone morphogenetic protein 4) in the second culture medium was 2 ng/mL.

### Example 3-2. Kit for inducing differentiation of human induced pluripotent stem cells into natural killer cells

In this example, provided is a kit for inducing the differentiation of human induced pluripotent stem cells into natural killer cells, similar to Example 1 but different in that the concentration of BMP4 (bone morphogenetic protein 4) in the second culture medium was 10 ng/mL.

### Example 3-3. Kit for inducing differentiation of human induced pluripotent stem cells into natural killer cells

In this example, provided is a kit for inducing the differentiation of human induced pluripotent stem cells into natural killer cells, similar to Example 1 but different in that the concentration of BMP4 (bone morphogenetic protein 4) in the second culture medium was 5 ng/mL.

### Example 4-1. Method for inducing differentiation of human induced pluripotent stem cells into natural killer cells

In this example, provided is a method for inducing the differentiation of human induced pluripotent stem cells into natural killer cells, similar to Example 2 but different in that the kit of Example 3-1 was used.

### Example 4-2. Method for inducing differentiation of human induced pluripotent stem cells into natural killer cells

In this example, provided is a method for inducing the differentiation of human induced pluripotent stem cells into natural killer cells, similar to Example 2 but different in that the kit of Example 3-2 was used.

### Example 4-3. Method for inducing differentiation of human induced pluripotent stem cells into natural killer cells

In this example, provided is a method for inducing the differentiation of human induced pluripotent stem cells into natural killer cells, similar to Example 2 but different in that the kit of Example 3-3 was used.

### Comparative Example 1. Kit for inducing differentiation of human induced pluripotent stem cells into natural killer cells

In this example, provided is a kit for inducing the differentiation of human induced pluripotent stem cells into natural killer cells, comprising: a first culture medium, a second culture medium, a third culture medium, a fourth culture medium, and a fifth culture medium.

The first culture medium was a StemFit^{®} Basic 03 medium containing 10 µM of Y27632 ·2HCl. The second culture medium was a differentiation medium containing 20 ng/mL of BMP4 (bone morphogenetic protein 4) and 3 µM of CHIR-99021; the differentiation medium was an RPMI1640 medium containing 1% (v/v) of non-essential amino acids, 1% (v/v) of glutamine, 50 µg/mL of vitamin C, and 2% (v/v) of vitamin A-free B27 medium supplement.

The third culture medium was a differentiation medium containing 5 ng/mL of BMP4, 50 ng/mL of VEGF-165 (vascular endothelial growth factor), 50 ng/mL of bFGF (basic fibroblast growth factor), and 10 µM of SB431542; the differentiation medium was an RPMI1640 medium containing 1% (v/v) of non-essential amino acids, 1% (v/v) of glutamine, 50 µg/mL of vitamin C, and 2% (v/v) of vitamin A-free B27 medium supplement.

The fourth culture medium was a differentiation medium containing 20 ng/mL of SCF (stem cell factor), 5 ng/mL of IL3 (interleukin 3), 10 ng/mL of IL15 (interleukin 15), 10 ng/mL of IL7 (interleukin 7), and 10 ng/mL of FLT3L (Fms-related tyrosine kinase 3 ligand); the differentiation medium was an X-VIVO15 medium containing 20% (v/v) of Human AB-FBS (human AB serum).

The fifth culture medium was a differentiation medium containing 20 ng/mL of SCF (stem cell factor), 10 ng/mL of IL15 (interleukin 15), 10 ng/mL of IL7 (interleukin 7), and 10 ng/mL of FLT3L (Fms-related tyrosine kinase 3 ligand); the differentiation medium was an X-VIVO15 medium containing 20% (v/v) of Human AB-FBS (human AB serum).

### Comparative Example 2. Method for inducing differentiation of human induced pluripotent stem cells into natural killer cells

In this example, provided is a method for inducing the differentiation of human induced pluripotent stem cells into natural killer cells, similar to Example 2 but different in that the kit of Comparative Example 1 was used and that the supernatant (suspended cells) in the six-well plates were collected into centrifuge tubes only on day 20 of the NK cell differentiation culture of the hemogenic endothelial cells in step (5).

### Comparative Example 3. Kit for inducing differentiation of human induced pluripotent stem cells into natural killer cells

In this example, provided is a kit for inducing the differentiation of human induced pluripotent stem cells into natural killer cells, comprising: a first culture medium, a second culture medium, a third culture medium, a fourth culture medium, and a fifth culture medium.

The first culture medium was a StemFit^{®} Basic 03 medium containing 10 µM of Y27632·2HCl. The second culture medium was a differentiation medium containing 20 ng/mL of BMP4 (bone morphogenetic protein 4) and 3 µM of CHIR-99021; the differentiation medium was an RPMI1640 medium containing 1% (v/v) of non-essential amino acids, 1% (v/v) of glutamine, 50 µg/mL of vitamin C, and 2% (v/v) of vitamin A-free B27 medium supplement.

The third culture medium was a differentiation medium containing 5 ng/mL of BMP4, 50 ng/mL of VEGF-165 (vascular endothelial growth factor), 50 ng/mL of bFGF (basic fibroblast growth factor), and 10 µM of SB431542; the differentiation medium was an RPMI1640 medium containing 1% (v/v) of non-essential amino acids, 1% (v/v) of glutamine, 50 µg/mL of vitamin C, and 2% (v/v) of vitamin A-free B27 medium supplement.

The fourth culture medium was a differentiation medium containing 20 ng/mL of SCF (stem cell factor), 5 ng/mL of IL3 (interleukin 3), 10 ng/mL of IL15 (interleukin 15), 10 ng/mL of IL7 (interleukin 7), 10 ng/mL of FLT3L (Fms-related tyrosine kinase 3 ligand), 5 ng/mL of sodium selenite, 50 µM of ethanolamine, 1 µM of β-mercaptoethanol, and 20 µg/mL of ascorbic acid; the differentiation medium was a DMEM/Ham's F-12(2:1)(DF-12) medium (DMEM high glucose medium:Ham's F-12 medium = 2:1(v/v)) containing 20% of Human AB-FBS (human AB serum).

The fifth culture medium was a differentiation medium containing 20 ng/mL of SCF (stem cell factor), 5 ng/mL of IL3 (interleukin 3), 10 ng/mL of IL15 (interleukin 15), 10 ng/mL of IL7 (interleukin 7), 10 ng/mL of FLT3L (Fms-related tyrosine kinase 3 ligand), 5 ng/mL of sodium selenite, 50 µM of ethanolamine, 1 µM of β-mercaptoethanol, and 20 µg/mL of ascorbic acid; the differentiation medium was a DMEM/Ham's F-12(2:1)(DF-12) medium (DMEM high glucose medium:Ham's F-12 medium = 2:1(v/v)) containing 20% of Human AB-FBS (human AB serum).

### Comparative Example 4. Method for inducing differentiation of human induced pluripotent stem cells into natural killer cells

In this example, provided is a method for inducing the differentiation of human induced pluripotent stem cells into natural killer cells, similar to Example 2 but different in that the kit of Comparative Example 3 was used and that the supernatant (suspended cells) in the six-well plates were collected into centrifuge tubes only on day 20 of the NK cell differentiation culture of the hemogenic endothelial cells in step (5).

### Comparative Example 5. Kit for inducing differentiation of human induced pluripotent stem cells into natural killer cells

In this example, provided is a kit for inducing the differentiation of human induced pluripotent stem cells into natural killer cells, similar to Example 1 but different in that the concentration of BMP4 (bone morphogenetic protein 4) in the second culture medium was 0 ng/mL.

### Comparative Example 6. Method for inducing differentiation of human induced pluripotent stem cells into natural killer cells

In this example, provided is a method for inducing the differentiation of human induced pluripotent stem cells into natural killer cells, similar to Example 2 but different in that the kit of Comparative Example 5 was used.

### Effect Example 1

The NK cells collected on days 13, 16, and/or 20 of the NK cell differentiation culture of the hemogenic endothelial cells in Example 2, Example 4-1, Example 4-2, Example 4-3, Comparative Example 2, Comparative Example 4, and Comparative Example 6 were collected into centrifuge tubes and centrifuged in a centrifuge at 1500 rpm for 5 min. The supernatant was discarded, and the cells were then resuspended in PBS. The cells resuspended in PBS were centrifuged again at 1500 rpm for 3 min, and the supernatant was discarded. Then, the cells were resuspended in 100 µL of PBS containing 0.5% bovine serum albumin (BSA), and a corresponding volume of the antibody was added according to the instructions for the antibody (the antibody is shown in Table 1). The cells were incubated in the dark at room temperature for 20 min, then washed three times with PBS, resuspended in 200 µL of PBS, and detected on a flow cytometer. The results are shown in FIGs. 2, 3, 4, 5, and 6: in Example 2, among the cells collected on day 13 of the NK cell differentiation culture of hemogenic endothelial cells, the proportion of CD45⁺/CD56⁺ cells was 72.18%; among the cells collected on day 16 of the NK cell differentiation culture of hemogenic endothelial cells, the proportion of CD45⁺/CD56⁺ cells was 89.53%; among the cells collected on day 20 of the NK cell differentiation culture for hemogenic endothelial cells, the proportion of CD45⁺/CD56⁺ cells was 96.72%, the proportion of CD45⁺/CD3⁻ cells was 98.87%, the proportion of CD56⁺/CD3⁻ cells was 97.12%, the proportion of CD56⁺/CD94⁺ cells was 53.45%, the proportion of CD45⁺/CD94⁺ cells was 47.90%, the proportion of CD3⁻/CD45⁺/CD56⁺ cells was 90.64%, the proportion of CD3⁻/CD16⁻ cells was 96.47%, the proportion of CD3⁻/Nkp46⁺ cells was 73.96%, the proportion of CD3⁻/CD56⁺/Nkp44⁺ cells was 93.35%, the proportion of CD3⁻/CD56⁺/Nkp30⁺ cells was 87.96%, and the proportion of CD3⁻/CD56⁺/Nkp44⁺/Nkp30⁺ cells was 85.77%; in Comparative Example 2, among the cells collected on day 20 of the NK cell differentiation culture of hemogenic endothelial cells, the proportion of CD45⁺/CD56⁺ cells was 95.10%, the proportion of CD45⁺/CD3⁻ cells was 99.24%, the proportion of CD43⁺/CD3⁻ cells was 97.95%, the proportion of CD56⁺/CD94⁺ cells was 29.37%, and the proportion of CD45⁺/CD94⁺ cells was 24.44%; in Comparative Example 4, among the cells collected on day 20 of the NK cell differentiation culture of hemogenic endothelial cells, the proportion of CD45⁺/CD56⁺ cells was 92.17%, the proportion of CD45⁺/CD3⁻ cells was 99.88%, the proportion of CD43⁺/CD3⁻cells was 99.14%, the proportion of CD56⁺/CD94⁺ cells was 14.92%, and the proportion of CD45⁺/CD94⁺ cells was 13.35%. It can be seen that the NK cell differentiation was substantially completed on day 20 of the NK cell differentiation culture of hemogenic endothelial cells; in Comparative Examples 2 and 4, among the cells collected on day 20 of the NK cell differentiation culture of hemogenic endothelial cells, the proportions of CD45⁺/CD56⁺ cells were slightly lower than that in Example 2, serum was detected in the fourth and fifth culture media in Comparative Examples 2 and 4, and two media were mixed to prepare the fourth and fifth culture media in Comparative Example 4, leading to complicated procedures. In Comparative Examples 2 and 4, among the cells collected on day 20 of the NK cell differentiation culture of hemogenic endothelial cells, the proportions of CD45⁺/CD94⁺ cells and CD56⁺/CD94⁺ cells were far less than those in Example 2, indicating a poor differentiation effect. Among the differentiated NK cells collected on day 20 of the NK cell differentiation culture of hemogenic endothelial cells at different concentrations of BMP4 (0 ng/mL, 2 ng/mL, 20 ng/mL, 10 ng/mL, and 5 ng/mL, corresponding to Comparative Example 6, Example 4-1, Example 2, Example 4-2, and Example 4-3, respectively), the proportions of CD45⁺/CD56⁺ cells were 34.95%, 58.86%, 96.72%, 90.29%, and 74.29%, respectively. It can be seen that 20 ng/mL of BMP4 is more favorable for NK cell differentiation. Similarly, the bright field cell observations of the differentiated NK cells collected on day 20 of the NK cell differentiation culture of hemogenic endothelial cells at different concentrations of BMP4 (0 ng/mL, 2 ng/mL, 10 ng/mL, and 5 ng/mL, corresponding to Comparative Example 6, Example 4-1, Example 4-2, and Example 4-3, respectively) are shown in FIG. 7: The cells at low concentrations (0-2 ng/mL) of BMP4 exhibited substantially no differentiation and no secretion in suspended cells, while the cells at the high concentration (10 ng/mL) of BMP4 exhibited secretion in suspended cells. It can be seen that 5-20 ng/mL of BMP4 is more conducive to NK cell differentiation.

**Table 1. Antibody information**

| Antibody | Fluorescent label | Source | Cat. No. |
|---|---|---|---|
| CD45 | PECY7 | Biolegend | 304016 |
| CD56 | PE | Biolegend | 304606 |
| CD3 | FITC | biolegend | 300306 |
| CD94 | FITC | BD Biosciences | 555888 |
| CD16 | APC | Biolegend | 360706 |
| Nkp44 | PECY7 | Biolegend | 325116 |
| Nkp30 | APC | Biolegend | 325210 |
| Nkp46 | APC | Biolegend | 331918 |

### Effect Example 2

The NK cells collected on day 20 of the NK cell differentiation culture of hemogenic endothelial cells in Example 2 were transferred to an IL2-containing medium (a basal medium containing 1 µM of UM171, 1 µM of SR1, 10 ng/mL of IL15, 20 ng/mL of SCF, and 50 ng/mL of IL2 (interleukin 2), wherein the basal medium was an IMDM medium containing 1% (v/v) of non-essential amino acids, 1% (v/v) of glutamine, 50 µg/mL of vitamin C, and 2% (v/v) of vitamin A-free B27 medium supplement) and cultured for 5 days. The NK (iPS-NK) cell cultures were collected into centrifuge tubes and centrifuged at 1500 rpm for 5 min in a centrifuge. The NK cell supernatant was collected into centrifuge tubes and detected according to the instructions of an IFN-γ ELISA kit (purchased from Stemcell, Cat. No. 7664-93-9). The secretion of IFN-γ was detected on a microplate reader (the supernatant was collected from peripheral blood-derived NK cells (PB-NK, Sailybio) treated with the same conditions (the post-treatment of PB-NK cells was completed with reference to the treatment of iPS-NK cells in this effect example: the NK cells were transferred to an IL2-containing medium and cultured for 5 days, the NK (iPS-NK) cell cultures were collected into centrifuge tubes and centrifuged at 1500 rpm for 5 min in a centrifuge, and the NK cell supernatant was collected into centrifuge tubes) as a control). The results are shown in FIG. 8: In Example 2, the concentration of IFN-γ secreted by the NK cells collected on day 20 of the NK cell differentiation culture of hemogenic endothelial cells after 5 days of the culture was 10,417.25 pg/mL, which was similar to the concentration of IFN-γ secreted by peripheral blood-derived NK (PB-NK) cells after the same treatment. It can be seen that the NK cells obtained by the present disclosure have a strong function of secreting IFN-γ.

### Effect Example 3

Tumor cells (Hela cells) with a cell density of 80% were digested and counted by AO/PI staining. The cells were seeded in a 96-well plate at 5 × 10⁴ cells/well and subjected to 5 h of adherent culture.

After 5 h, the NK cells collected on day 20 of the NK cell differentiation culture of hemogenic endothelial cells in Example 2 were transferred to the IL2-containing medium described in Effect Example 2 and cultured for 5 days. The NK cell cultures (IMDM-iNK) were collected into centrifuge tubes (peripheral blood-derived NK cells (PB-NK, Sailybio) treated with the same conditions (the culture of NK cells in the IL2-containing medium described in Effect Example 2) were used as a control) and centrifuged at 1500 rpm for 5 min in a centrifuge. The supernatant was discarded, and then the cells were resuspended in the IL2-containing medium of Effect Example 2 and counted by AO/PI staining. The counted NK cells and tumor cells were seeded in different (cancer cell:NK cell) ratios of 1:0, 1:1, 1:3, 1:5, and 1:10. After the cells were co-incubated for 18 h, the supernatant was discarded by pipetting, and the fresh IL2-containing culture medium described in Effect Example 2 was added at 100 µL/well. 10 × CCK8 was added to the culture medium in advance, and after the cells were incubated in an incubator at 37 °C and 5% CO ₂ for 1.5 h, the 96-well plate was taken out of the incubator, and the fluorescence values were detected on a microplate reader to determine the killing effect of NK cells on tumor cells. The results are shown in FIG. 9: When the ratios of cancer cells to NK cells were 1:1, 1:3, 1:5, and 1:10, the killing rates of Hela cells by the NK cells in Example 2 were 17%, 45%, 67%, and 80%, respectively, which were comparable to the killing rates by peripheral blood-derived NK cells (PB-NK) (33%, 57%, 76%, and 76%). It can be seen that the NK cells obtained by the present disclosure have a good function of killing tumor cells.

### Effect Example 4

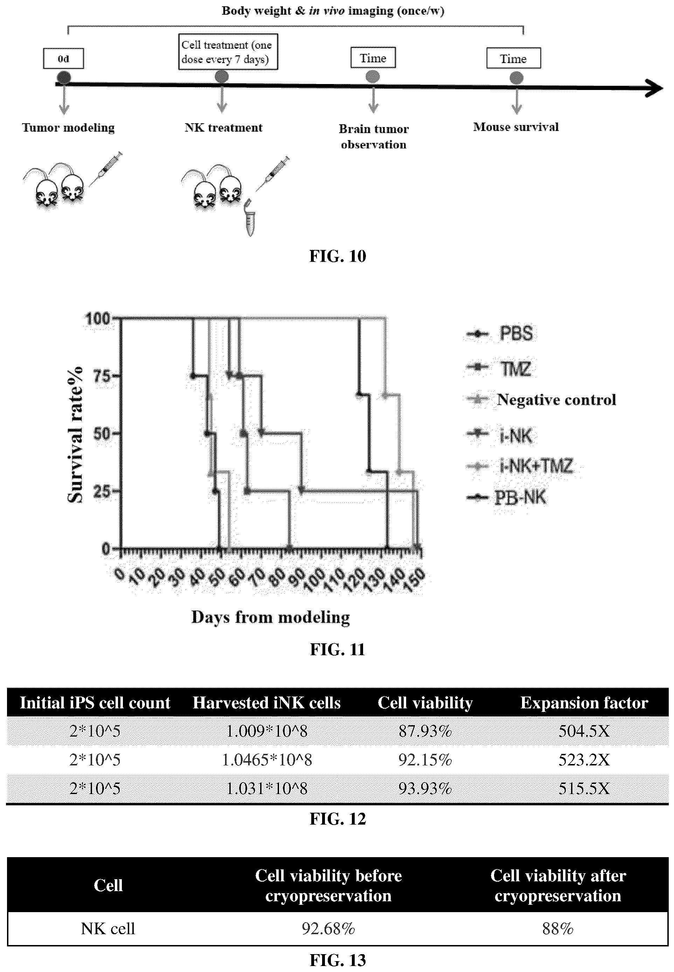

A schematic flow chart of this effect example is shown in FIG. 10. Specifically, 1.3 × 10⁵ to 1.5 × 10⁵ U87 glioma cells were injected into mice 5 days in advance (D-5) to establish a U87 glioma cell mouse tumor model. After the successful establishment of the mouse tumor model (D0), the mice were divided into 6 groups of 4 mice, each receiving three doses, specifically: 1) the PBS group (tumor model mice receiving an equal volume of PBS), 2) the TMZ group (tumor model mice receiving temozolomide, once every seven days at 5 mpk/dose, positive control), 3) the negative control group (tumor model mice receiving an equal volume of PBS containing 10 µM of IL15 and 20 µM of IL12, once every seven days), 4) the i-NK group (tumor model mice receiving iPS-NK cells at 5 × 10⁵/µL per mouse, once every seven days at 5 µL/dose, and the i-NK cells were prepared as follows: the NK cell cultures collected on day 20 of the NK cell differentiation culture of hemogenic endothelial cells in Example 2 were collected in centrifuge tubes and centrifuged at 1500 rpm for 5 min in a centrifuge; the supernatant was discarded, and then the cells were resuspended in 10 mL of PBS containing 10 µM of IL15 and 20 µM of IL12; the cell suspension was sieved through a 40 µM cell strainer to ensure that the cells were in a single cell state; the cells were centrifuged at 1500 rpm for 5 min in a centrifuge; the supernatant was discarded, and the cells were then resuspended in 5 mL of PBS containing 10 µM of IL15 and 20 µM of IL12 and counted by AO/PI staining), 5) the PB-NK group (tumor model mice receiving PB-NK cells at 5 × 10⁵/µL per mouse, once every seven days at 5 µL/dose, and the PB-NK cells were prepared as follows: the peripheral blood-derived NK (PB-NK) cell cultures were collected in centrifuge tubes and centrifuged at 1500 rpm for 5 min in a centrifuge; the supernatant was discarded, and then the cells were resuspended in 10 mL of PBS containing 10 µM of IL15 and 20 µM of IL12; the cell suspension was sieved through a 40 µM cell strainer to ensure that the cells were in a single cell state; the cells were centrifuged at 1500 rpm for 5 min in a centrifuge; the supernatant was discarded, and the cells were then resuspended in 5 mL of PBS containing 10 µM of IL15 and 20 µM of IL12 and counted by AO/PI staining), and 6) i-NK + TMZ treatment group (tumor model mice receiving i-NK cells at 5 × 10⁵/µL and TMZ at 5 mpk, once every seven days at 5 µL/dose). The survival results of mice are shown in FIG. 11: Basically all mice in the PBS control group and the negative control group died around D40-D50; the survival rate of mice in the i-NK group was significantly higher than that in the TMZ group but slightly lower than that in the natural NK cell group (PB-NK group), and the survival rate of mice in the i-NK + TMZ combination group was significantly superior to that in the natural NK group. In addition, it should be noted that when i-NK cells were used alone, 1 of 4 mice in the group survived until the end of study (D150), indicating that the mouse was substantially cured. It can be seen that the NK cells prepared by the present disclosure exhibited a good anti-tumor effect, and exhibited a synergistic effect when used in combination with other anti-tumor drugs, thereby further enhancing the anti-tumor effect of the NK cells prepared by the present disclosure.

### Effect Example 5

In the differentiation process of the stem cells into the hemogenic endothelial cells, low-attachment 96-well plates were used to ensure the uniformity of spherical EBs and thereby improve the differentiation efficiency. According to the method of Example 2, iPSCs were seeded at an initial amount of 2 × 10⁵ cells/well in 96-well plates, and 1.009 × 10⁸ to 1.0465 × 10⁸ NK cells were finally harvested after the differentiation (NK cells were collected on day 20 of the NK cell differentiation culture of hemogenic endothelial cells, and the results are shown in FIG. 12. From the initial iPSCs to the finally harvested mature NK cells, the cells were expanded by more than 500 folds, thus requiring no conventional NK cell expansion procedures and greatly facilitating the cell transplantation and disease treatment). Additionally, the NK cells cryopreserved with the cryopreservation solution (CryoStor^{®} CS10; purchased from Stemcell Technologies; the cell concentration was 1 × 10⁷ cells/mL, i.e., 1 mL of cryopreservation solution contained 1 × 10⁷ cells; the cryopreservation method was that the cryopreservation solution containing the cells was frozen to -80 °C by a programmed box freezer, preserved in a freezer at -80 °C for 24 h, and then preserved in a liquid nitrogen tank for 1 week) still exhibited good cell viability (FIG. 13).

### Example 5. Sensitivity response of different GBM cell strains to TMZ

Clinically, GBM tumor cells may exhibit sensitivity or resistance to TMZ. In this example, the evaluation of sensitivity response to TMZ was conducted on different GBM tumor cell strains. TMZ-sensitive GBM (U87) and TMZ-resistant GBM (T98G) were purchased from Zhejiang Meisen Cell Technology Co., Ltd., and U87 and T98G cells were cultured in a DMEM medium (Gibco, 11965084) containing 10% of FBS (Cellmax, SA211.02).

### 5.1. Sensitivity response of GBM to TMZ at different concentrations

### A. Cell plating

U87 and T98G cells were cultured until an 80%-90% confluence and digested with pancreatin (Beyotime, C0201-100mL) at 37 °C for 5 min. DMEM medium (Gibco, 11965084) of a two-fold volume was added to stop the digestion. The cell suspension was transferred to a centrifuge tube and centrifuged at 300 g for 5 min. The cell pellet was resuspended in DMEM medium, and the cells were counted by AO (acridine orange)/PI (propidium iodide) staining. According to the counting result, the density of the cell suspension was adjusted to 1 × 10⁵ cells/mL. The cell suspension was added to the 96-well plate at 100 µL/well as required for grouping, and then the 96-well plate was incubated in an incubator at 37 °C overnight.

### B. TMZ preparation

TMZ powder (Sigma, T2577-25MG) was dissolved in an appropriate volume of DMSO to prepare a TMZ stock solution at a concentration of 100 mM. The TMZ solution with the highest concentration (5000 µM) was prepared as follows: 225 µL of the TMZ stock solution was diluted with 4.5 mL of DMEM medium, and the resultant solution was then diluted to give TMZ working solutions at concentrations of 1000 µM, 500 µM, 200 µM, 100 µM, and 10 µM.

### C. TMZ Treatment

After the U87 and T98G cells were cultured overnight, and the adherent growth and the logarithmic growth phase were observed, the TMZ working solutions at the corresponding working concentration were added according to the grouping design at 100 µL/well, and the cells were incubated in the incubator. After 72 h, the cell viability and count were determined.

### D. Cell viability assay

The CellTiter-Glo^{®} luminescent cell viability assay kit (Promega, G7572) was used, and the procedures in the manufacturer's instructions were performed. An appropriate volume of the CellTiter-Glo^{®} solution was mixed with a DMEM medium in a ratio of 1:1 to prepare a working solution. The CellTiter-Glo^{®} working solution was added to the 96-well plate at 50 µL/well. The plate was incubated at room temperature for 10 min in the dark and then detected on a microplate reader (TECAN, Infinite^{®}200 PRO) for the chemiluminescence.

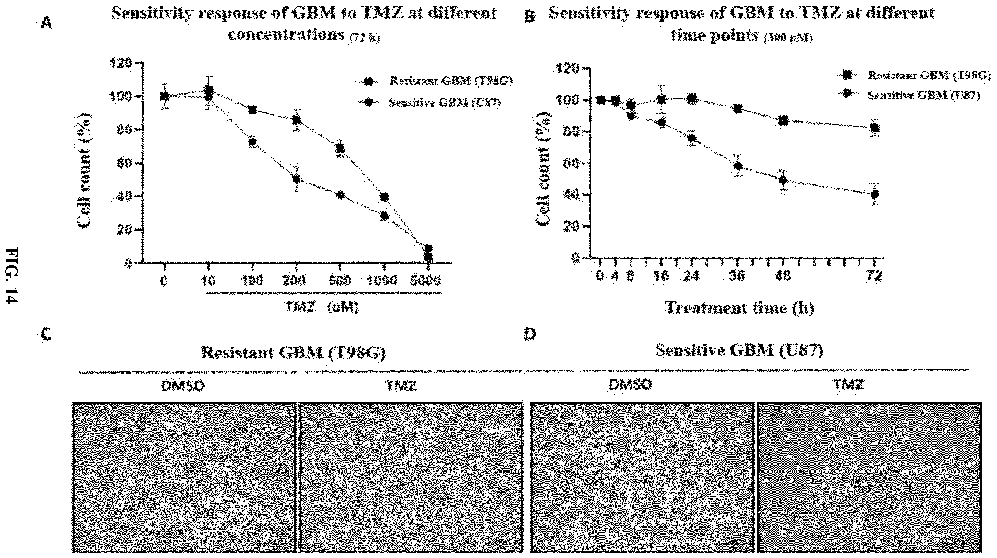

The detection results are shown in FIG. 14(A). With the increase of the TMZ concentration, the U87 cell count was significantly reduced, suggesting a sensitive response, while the T98G cell count began to significantly reduce over 500 µM, suggesting an insensitive response. When TMZ was at 200 µM, the viable U87 cell count was about 50%, while the viable T98G cell count remained at 80% or higher; when TMZ was at 500 µM, the viable U87 cell count was about 40%, while the viable T98G cell count remained at 70%. The sensitivity difference interval was 100 µM to 500 µM.

### 5.2. Sensitivity response of GBM to TMZ over different periods of time

The assay and detection schemes were the same as those in Example 5.1, and a concentration of 300 µM in the sensitive interval in Example 5.1 was selected as the TMZ concentration.

The detection results are shown in FIG. 14(B). With the increase in time of the TMZ treatment, the viable U87 cell count was significantly and gradually reduced to less than 40% at 72 h; T98G was less affected by TMZ, and the viable cell count remained above 80% at 72 h. The above results indicate that U87 is TMZ-sensitive GBM and T98G is TMZ-resistant GBM. FIG. 14(C) shows the microscopic results of the resistant GBM (T98G) cells treated with TMZ (300 µM) for 48 h, and FIG. 14(D) shows the microscopic results of the sensitive GBM (U87) cells treated with TMZ (300 µM) for 48 h. The microscopic results show that after 48 h of treatment with TMZ (300 µM), the growth of T98G cells was not greatly different compared to the control DMSO treatment, while the growth of U87 cells was significantly inhibited.

### Example 6. Resistance to TMZ-induced cell cycle arrest in resistant GBM (T98G)

TMZ acts on DNA after being absorbed into tumor cells in the human body, causing alkylation damage to the DNAs, thereby forming DNA double-strand breaks or cross-linking, arresting the cell cycle, and triggering the proliferation arrest or apoptosis of the tumor cells. To explore the mechanism by which the resistant GBM cell strains resist TMZ, a cell cycle assay was performed to determine the effect of TMZ on the cell cycle of different GBM cell strains.
A. Cell culture and treatment: See Example 5 for the procedures of U87 and T98G cell culture and TMZ treatment. The concentration of TMZ used in this example was 300 µM.
B. Preparation of cell samples: The Beyotime cell cycle kit (Beyotime, C1052) was used according to the manufacturer's instructions. 1 × 10⁶ cells were added to a centrifuge tube for later use and centrifuged at 1000 g for 3-5 min to precipitate the cells. The supernatant was discarded by pipetting, and 1 mL of pre-cooled PBS was added to resuspend the cells. The mixture was transferred to a 1.5 mL centrifuge tube. The cells were pelleted by centrifugation, and the supernatant was discarded by pipetting.
C. Cell fixation: The cells were added to 1 mL of pre-cooled 70% ethanol, mixed well by pipetting, and fixed at 4 °C for 30 min. The mixture was centrifuged at about 1000 g for 3-5 min to pellet the cells. The supernatant was discarded by pipetting, and about 1 mL of PBS pre-cooled in an ice bath was added to resuspend the cells. The cells were pelleted again by centrifugation, and the supernatant was discarded by pipetting.
D. Preparation of and staining with propidium iodide (PI) staining solution:
Referring to Table 2, an appropriate amount of PI staining solution was prepared according to the number of samples to be tested.

**Table 2. Preparation of PI staining solutions**

| | 1 sample | 6 samples | 12 samples |
|---|---|---|---|
| Staining buffer | 0.5 mL | 3 mL | 6 mL |
| Propidium iodide staining solution (20×) | 25 µL | 150 µL | 300 µL |
| RNase A (50X) | 10 µL | 60 µL | 120 µL |
| Final volume | 0.535 mL | 3.21 mL | 6.42 mL |

| | | | |
|---|---|---|---|
| Note: In this example, the PI staining solution was prepared as per 12 samples. | | | |

Staining: 0.5 mL of the PI staining solution was added to each tube of cell samples, and the cell pellet was slowly and fully resuspended and incubated in the dark at 37 °C for 30 min. The mixture was preserved at 4 °C or in an ice bath in the dark. The flow cytometry was completed within 24 h after the staining was completed.

E. Flow cytometry detection and analysis: The red fluorescence was detected at an excitation wavelength of 488 nm and the light scattering was also detected on a flow cytometer (Agilent, Novocyte3000). The cellular DNA content assay and light scattering analysis were performed using an appropriate analysis software (Agilent, Novoexpress).

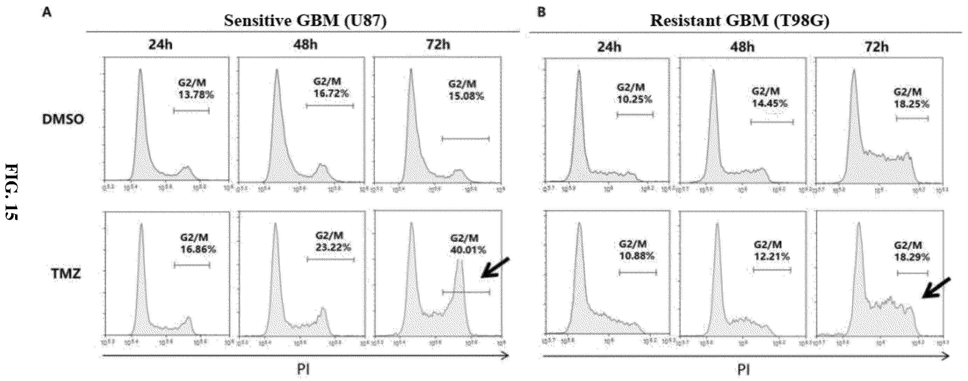

Upon entering the nucleus, PI binds to DNA and generates fluorescence. The intensity of PI is positively correlated with the amount of DNA in the cell. The peak on the left in each flow cytometric diagram denotes the DNA at 2 N in the G1 phase of the cell cycle, the peak on the right denotes the DNA at 4 N in the G2/M phase of the cell cycle, and the middle part denotes the DNA being synthesized in S phase (between 2N-4N). As shown in FIG. 15(A), for the sensitive GBM (U87), after the TMZ treatment, the DNA in the G2/M phase gradually accumulated over time, and compared with the control DMSO-treated cells, the DNA content in the G2/M phase was increasing (16.86% at 24 h, 23.22% at 48 h, and 40.01% at 72 h), indicating that the cell cycle was arrested in the G/M phase; in contrast, as shown in FIG. 15(B), for the resistant GBM (T98G), after the TMZ treatment, the DNA in the G2/M phase did not change significantly, and the DNA content was similar to that of the control DMSO-treated group. The above data indicate that TMZ can cause cell cycle arrest in GBM cells, and resistant GBM (T98G) can resist such cell cycle arrest.

### Example 7. TMZ-induced up-regulation of a series of immune activation-related ligand expression in resistant GBM tumor cells

Immune surveillance is an important mechanism for identifying and removing tumor cells, and the dynamic expression of proteins related to immune activation on the membrane of tumor cells is also the key to their recognition by immune cells or recognition escape. In this example, the changes in expression of immune-related proteins when GBM is resisting the effect of TMZ were further studied, so as to provide a theoretical basis for the immunotherapy.
A. GBM cell culture and treatment: See Example 5 for the procedures of T98G cell culture. The cells were cultured until an 80%-90% confluence and digested with pancreatin at 37 °C for 5 min. DMEM medium of a two-fold volume was added to stop the digestion. The cell suspension was transferred to a centrifuge tube and centrifuged at 300 g for 5 min. The cell pellet was resuspended in DMEM medium, and the cells were counted by AO/PI staining. According to the counting result, the density of the cell suspension was adjusted to 1 × 10⁵ cells/mL. After that, an appropriate volume of TMZ stock solution (100 mM) was added to the cell suspension to reach a final TMZ concentration of 1000 µM in the cell suspension. The cells were seeded into a 24-well plate at 500 µL/well.
B. Incubation and flow cytometry of antibodies for immune-related ligands

The cells treated with TMZ for 48 h were digested with a pancreatin digestion solution and centrifuged, and the supernatant was discarded. The corresponding antibodies as shown in Table 3 were added, and the cells were incubated at room temperature for 30 min. The cells were washed with PBS, centrifuged, resuspended, and detected on a flow cytometer.

**Table 3. Antibodies against immune activation-related targets**

| No. | Target | Fluorescence channel | Manufacturer | Cat. No. |
|---|---|---|---|---|
| 1 | CD112 | PE | Biolegend | 337409 |
| 2 | CD155 | PE | Biolegend | 337609 |
| 3 | B7-H6 | PE | R&D | FAB7144P-025 |
| 4 | CD54 | PE | Biolegend | 353105 |
| 5 | CD70 | PE | Biolegend | 355103 |
| 6 | CD48 | PE | Biolegend | 336707 |
| 7 | CD1d | APC | Biolegend | 350307 |
| 8 | PD-L1 | BV421 | Biolegend | 374508 |

| Isotype control in each group | | | | |
|---|---|---|---|---|
| 9 | Mouse IgG2A PE-conjugated Antibody | PE | R&D | IC003P |
| 10 | PE Mouse IgGl, κ Isotype Ctrl | PE | Biolegend | 400114 |
| | (FC) Antibody | | | |
| 11 | Mouse IgG1 PE-conjugated Antibody | PE | R&D | IC002P |
| 12 | APC Mouse IgG2b, κ Isotype Ctrl Antibody | APC | Biolegend | 400319 |
| 13 | Brilliant Violet 421^{™} Mouse IgG1, κ Isotype Ctrl Antibody | BV421 | Biolegend | 400158 |

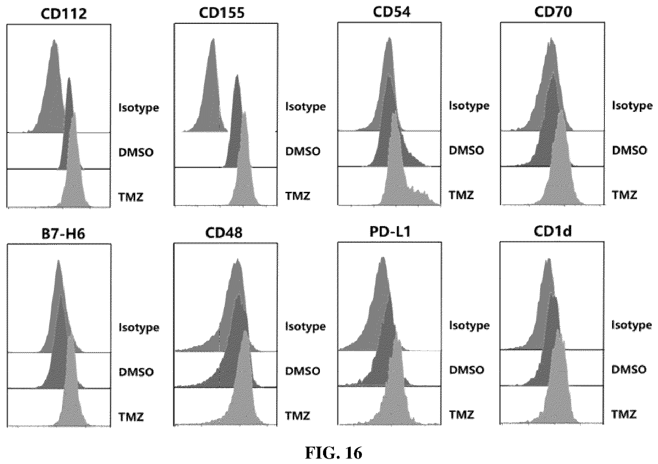

Immune activation ligands against GBM tumor cells are important targets for immune cell surveillance and can even activate NK cells. As shown in FIG. 16, the flow cytometry results show that various immune activation ligands exhibited up-regulated expression to different extents after 48 h of TMZ treatment. The above data indicate that the up-regulated expression of a series of immune activation ligands may induce stronger NK killing effects, providing a theoretical basis for the treatment of resistant GBM with TMZ in combination with immune cells.

### Example 8. TMZ-induced up-regulation of GBM immune-related ligand expression and resultant stronger immune response of iPSC-NK

The effect of NK cells in combination with TMZ on GBM tumor cells was further determined in an *in vitro* killing study to explore the possibility of the combination therapy. The NK cells used in this example were human induced pluripotent stem cell (iPSC)-derived NK cells.

### 8.1. Direct induction of differentiation of human induced pluripotent stem cells (iPSCs) to NK cells in vitro

In this example, the human induced pluripotent stem cells (iPSCs) used were prepared from human peripheral blood cells using the CTS^{™} CytoTune^{™}-iPS 2.1 Sendai Reprogramming Kit (Cat. No. A34546) according to the manufacturer's instructions, which, however, does not limit the human induced pluripotent stem cells and the preparation thereof in the present disclosure. Human induced pluripotent stem cells commonly known in the art and methods for preparing the same may be encompassed.
A. The iPSCs were cultured in a low-attachment 96-well plate to give embryoid bodies. 2 × 10⁵ iPSCs were evenly distributed in a 96-well plate, seeded in a low-attachment 96-well plate (Corning, 3474) with the StemFit Basic 03 medium (Ajinomoto, StemFitBasic03Liquid A/B/rHu BFGF; first culture medium) containing 10 µM of Y27632 ·2HCl (Selleck, S6390), and cultured for 1 day to give embryoid bodies.
B. Mesoderm differentiation. After 1 day, the medium was changed to a mesoderm differentiation medium, and the cells were cultured for 2 days.
   Mesoderm differentiation medium (second culture medium): RPMI1640 (Gibco, 22400-089) supplemented with 1% of non-essential amino acids (Gibco, 11140050), 1% of glutamine (Gibco, 35050-061), 50 µg/mL of vitamin C (Sigma, S4226), 2% (v/v) of a vitamin A-free B27 medium supplement (Gibco, 12587-010), 20 ng/mL of BMP4 (R&D, 314-BP), and 3 µM of CHIR-99021 (Selleck, S1263).
C. Hemogenic endothelial cell differentiation. After 2 days, the mesoderm differentiation medium was replaced with a hemogenic endothelium differentiation medium, and the cells were cultured for 2 days.
   Hemogenic endothelium differentiation medium (third culture medium): RPMI1640 (Gibco, 22400-089) supplemented with 1% of non-essential amino acids (Gibco, 11140050), 1% of glutamine (Gibco, 35050-061), 50 µg/mL of vitamin C (Sigma, S4226), 2% (v/v) of a vitamin A-free B27 medium supplement (Gibco, 12587-010), 5 ng/mL of BMP4 (R&D, 314-BP), 50 ng/mL of VEGF (Novoprotein, C083), 50 ng/mL of bFGF (Sino Biological, 10014-HNAE), and 10 µM of SB431542 (Selleck, S1067).
D. NK cell differentiation-1. After 2 days, the embryoid bodies in the low-attachment 96-well plate were transferred into a 50 mL centrifuge tube, and the cells were allowed to settle naturally for 1-2 min. The supernatant was discarded until the remaining supernatant in the centrifuge tube was about 10 mL. Then about 40 mL of NK cell differentiation medium 1 was added. The cell pellet was mixed well by gently pipetting and transferred to a T225 culture flask for culture for 7 days.
   NK cell differentiation medium 1 (fourth culture medium): IMDM medium (Gibco, 12400-053) supplemented with 1% of non-essential amino acids (Gibco, 11140050), 1% of glutamine (Gibco, 35050-061), 50 ng/mL of vitamin C (Sigma, S4226), 2% (v/v) of a vitamin A-free B27 medium supplement (Gibco, 12587-010), 5 ng/mL of BMP4 (R&D, 314-BP), 10 ng/mL of VEGF (Novoprotein, C083), 20 ng/mL of SCF (Novoprotein, GMP-CD53), 5ng/mL of IL3 (PeproTech, 200-03), 10 ng/mL of IL15 (Sino Biological, GMP-10360-HNCE), 20 ng/mL of IL7 (Sino Biological, GMP-11821-HNCE), and 10 ng/mL of FLT3L (PeproTech, 300-19).
E. NK cell differentiation-2. After 7 days of culture, NK cell differentiation medium 2 was added, and the cells were cultured for another 7 days.
   NK cell differentiation medium 2 (fifth culture medium): IMDM medium (Gibco, 12400-053) supplemented with 1% of non-essential amino acids (Gibco, 11140050), 1% of glutamine (Gibco, 35050-061), 50 µg/mL of vitamin C (Sigma, S4226), a vitamin A-free B27 medium supplement (Gibco, 12587-010), 5 ng/mL of BMP4 (R&D, 314-BP), 10 ng/mL of VEGF (Novoprotein, C083), 20 ng/mL of SCF (Novoprotein, GMP-CD53), 10 ng/mL of IL15 (Sino Biological, 10360-HNCE), 20 ng/mL of IL7 (Sino Biological, 11821-HNCE), 10 ng/mL of FLT3L (PeproTech, 300-19), 1 µM of UM171 (APExBIO, A8950), 1µM of SR1 (Selleck, S2858), and 30 µM of NAC (Sigma, A9165).
F. NK cell differentiation-3. After 7 days of culture, NK cell differentiation medium 2 was added again, and the cells were cultured for another 7 days.
G. NK cell activation and expansion. The differentiated NK cells were collected and centrifuged in a centrifuge at 1500 rpm for 10 min, and resuspended in 5 mL of an NK cell activation and expansion medium. The cell suspensions were then filtered through a 70 µm cell strainer. The filtered cell suspensions were centrifuged in a centrifuge again at 1500 rpm for 5 min, and resuspended in 5 mL of the NK cell activation and expansion medium. The initial density was adjusted to 1 ×10⁶ cells/mL, and the medium was replaced once every 3-4 days. The cells were cultured for 7-10 days and collected for subsequent functional assays.

Activation and expansion medium: CTS NK-Xpander medium (GIBCO, A5019001), supplemented with 500 IU of IL-2 (PeproTech, 200-02), 10 ng/mL of IL-15 (Sino Biological, 10360-HNCE), 10 ng/mL of IL-18 (Sino Biological, 10119-HNCE), and 5% of hAB (Shanghai Hengyuan Biological Technology, HY120-AB).

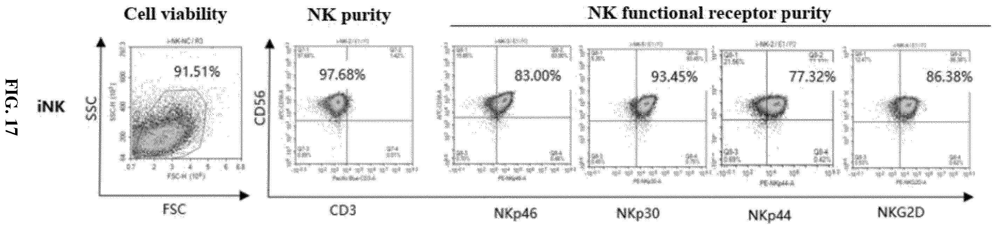

The harvested iPSC-NK (iNK) cells were finally characterized by flow cytometry. The characterization results are shown in FIG. 17. The *in vitro* directionally differentiated NK cells from the iPSCs exhibited ideal cell viability (91.51%), purity (97.68%), and expression of functional receptors (purity > 75%). Information on antibodies related to NK cell characterization is shown in Table 4.

**Table 4. Antibodies against immune activation-related targets**

| Antibody target | Fluorescence channel | Manufacturer | Cat. No. |
|---|---|---|---|
| CD3 | BV421 | Biolegend | 317344 |
| CD56 | APC | BD Biosciences | 555518 |
| NKG2D | PE | Biolegend | 320806 |
| NKp30 | PE | Biolegend | 325208 |
| NKp46 | PE | BD Biosciences | 557991 |
| NKp44 | PE | BD Biosciences | 558563 |

### 8.2. Higher degranulation activity of iPSC-NK in combination with TMZ

Changes in immune response intensity (degranulation activity) were determined by co-incubation of the iPSC-NK cells differentiated in Example 8.1 with GBM tumor cells or with TMZ-treated GBM tumor cells. The assay design is shown in Table 5.

**Table 5. Design of degranulation activity assay**

| Target cell | Group | | | |
|---|---|---|---|---|
| TMZ-resistant GBM | iNK | iNK+T98G | iNK+TMZ+T98G | iNK+PMA/io |
| TMZ-sensitive GBM | iNK | iNK+U87 | iNK+TMZ+U87 | iNK+PMA/io |

Preparation of target cells: For the procedures T98G and U87 culture, see Example 5. The target cells in the TMZ pretreatment group were treated with 1000 µM TMZ for 48 h in advance for later use.

Plating of target cells: The target cells in each group were centrifuged in advance, resuspended, counted, diluted to a density of 5 × 10⁵ cells/mL, and seeded in corresponding plates at 100 µL/well according to the grouping design.

Administration of effector cells: The iNK cells were centrifuged, resuspended, counted, and diluted to a cell density of 5 × 10⁵ cells/mL. The effector cells were seeded in corresponding plates at 100 µL/well and mixed well by gently pipetting.

Blank control: 100 µL of the activation and expansion medium was supplemented in the blank control wells.

Positive control: PMA/io (Biolegend, 423301) was added to 5 mL of activation and expansion medium in a ratio of 1:500. The mixture was mixed well by pipetting and added to positive control wells at 100 µL/well.

Incubation with CD107a antibody (BD, 560664): An antibody suspension was prepared at 1 µL/well, diluted with the activation and expansion medium 10-fold in volume, mixed well by pipetting, and added at 10 µL/well.

The prepared plates were incubated in an incubator for 1 h.

Addition of Golgi blockers: The GolgiStop (BD, 51-2092KZ) and GolgiPlug (BD, 51-2301KZ) were diluted in a complete medium 10-fold in volume at 0.2 µL/well, and added to the corresponding wells at 10 µL/well.The plates were incubated in an incubator for 3 h.

After the incubation was completed, the cells in the plates were transferred to corresponding 1.5 mL centrifuge tubes and centrifuged at 800 g for 5 min. The supernatant was discarded, and the cells were resuspended in 100 µL of PBS. 1 µL of CD56 flow cytometry antibody was added to each tube, and the system was incubated at room temperature in the dark for 20 min and centrifuged at 800 g for 5 min. The supernatant was discarded, and the cells were resuspended in 100 µL of PBS and subjected to flow cytometry.

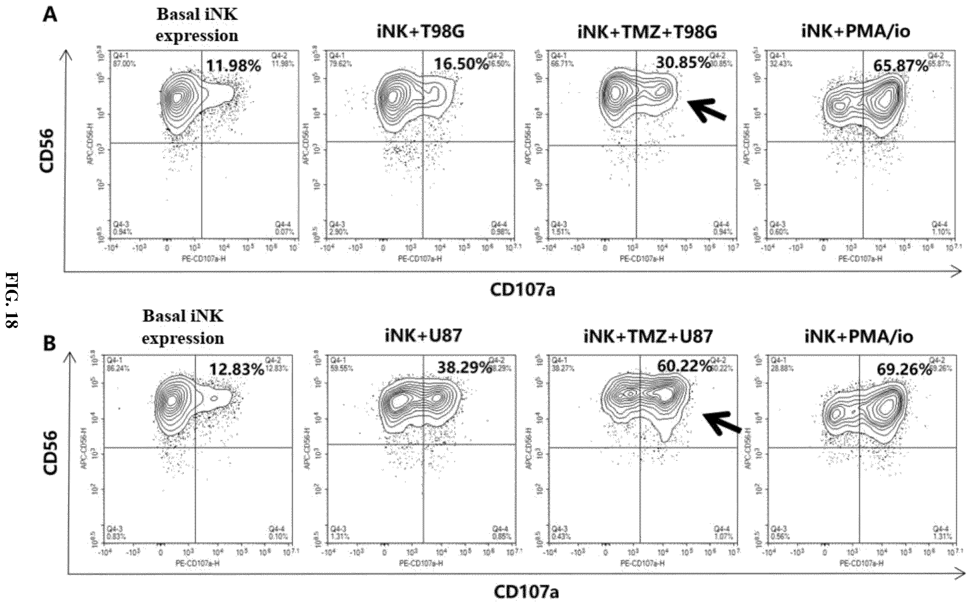

As shown in FIG. 18(A), for the resistant strain T98G, the co-incubation of iNK cells with target cells induced the activation of degranulation in a certain proportion, with the expression of CD107a increasing from 11.98% of the basal level to 16.50%, whereas, for the combination with TMZ, the expression of CD107a was significantly improved to 30.85%, indicating that iNK cells combined with TMZ enhanced the immune activation ability of NK cells.

As shown in FIG. 18(B), for the sensitive strain U87, the co-incubation of iNK cells with target cells induced an activation of degranulation in a higher proportion, with the expression of CD107a increasing from 12.83% of the basal level to 38.29%, whereas, for the combination with TMZ, the expression of CD107a was further improved to 60.22%.

The above results indicate that TMZ can induce a stronger immune response in iNK cells to GBM tumor cells.

### Example 9: Enhanced tumor cell killing activity of iPSC-NK cells in combination with TMZ on resistant GBM (T98G)

Furthermore, in this example, the *in vitro* efficacy of iPSC-NK cells in combination with TMZ was explored by monitoring the killing of tumor target cells.
A. The procedures for the culture, digestion, and plating of GBM tumor cells T98G are described in Example 5.
B. TMZ preparation: See Example 5 for the procedures of treating cells. In this example, the cells were pre-treated at 1000 µM for 48 h.
C. iPSC-NK cells were prepared as described in Example 8.
D. Co-incubation for killing. According to the preset effector-to-target ratios (1:8, 1:4, and 1:1), an appropriate volume of iNK cells in an expansion culture was taken (note that the iNK cell viability should be kept at 90% or higher) and counted by AO/PI staining. According to the counting result, the cell suspension was adjusted to a density of 1 × 10⁵ cells/mL. The tumor cells were seeded at 1 × 10⁴ cells/well. After the adherent culture of T98G cells in the 96-well plates (6 h after re-digestion and inoculation), the NK cell suspension was added to the T98G plates according to the grouping design. After 18 h of co-incubation, the viability of the T98G cells was detected using the CellTiter-Glo^{®} luminescent cell viability assay kit. Three replicates were set in each group.
E. Cell viability assay. At preset time points (8, 16, 24, and 48 h), the cells were collected. The CellTiter-Glo^{®} luminescent cell viability assay kit was used according to the manufacturer's instructions. An appropriate volume of the CellTiter-Glo^{®} solution was mixed with a DMEM medium in a ratio of 1:1 to prepare a working solution. The CellTiter-Glo^{®} working solution was added to the 96-well plate at 50 µL/well. The plate was incubated at room temperature for 10 min in the dark and then detected on a microplate reader for the chemiluminescence.
F. Killing rate calculation. Killing rate (%) = (fluorescence intensity in the untreated group - fluorescence intensity in the treatment group)/fluorescence intensity in the untreated group × 100%. * denotes p < 0.05 in a T test, and ** denotes p < 0.01.

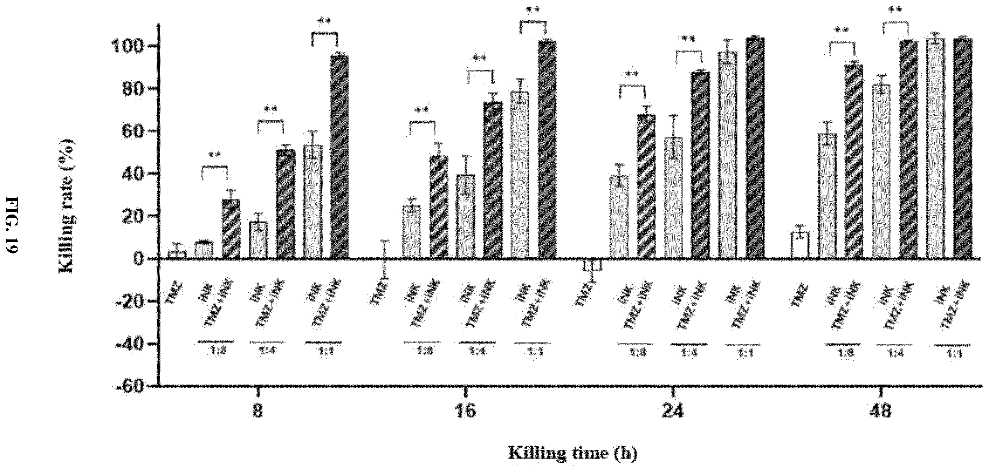

The results, as shown in FIG. 19, indicate that TMZ exhibited a weak killing effect against the resistant GBM (T98G) after 8 h, 16 h, 24 h, and 48 h of co-incubation, and a killing effect of only about 10% was observed at 48 h (blank bars). iNK cells exhibited a significant killing effect against target cells and an increase over time as the effector-to-target ratio increases (gray bars). Importantly, the killing against target cells in the iNK + TMZ combination group was significantly improved at the corresponding time points and in various effector-to-target ratios (black slash bars). The above results show that although the resistant GBM (T98G) cells possess resistance to TMZ, the combination of TMZ with iNK cells exhibited potent tumor killing activity against the tumor cells.

### Example 10: Synergistic effect of NK cells of other sources (NK92, PB-NK) in combination with TMZ on resistant GBM

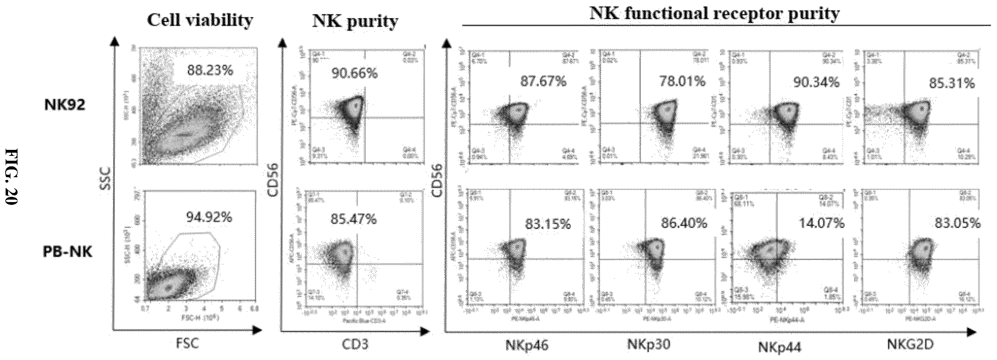

Given that immune target recognition and immune tumor killing are basic immune mechanisms against tumors, the source of NK cells is not limited to iPSC-NK, and NK cells of other sources were also used in this example for in-depth study and comparison. The NK92 cells were purchased from Zhejiang Meisen Cell Technology Co., Ltd., and the peripheral blood-derived PB-NK cells were purchased from Shanghai Hycells Biotechnology Co., Ltd.
A. NK92 cell expansion culture. NK92 medium: CTS NK-Xpander medium (GIBCO, A5019001), supplemented with 500 IU of IL-2 (PeproTech, 200-02) and 5% of hAB (Shanghai Hengyuan Biological Technology, HY120-AB). The cells were seeded at a density of 2 × 10⁵ to 5 × 10⁵ cells/mL, and the medium was half-exchanged once every 48 h. The cells were incubated in an incubator at 37 °C.
B. PB-NK cell expansion culture. PB-NK medium: CTS NK-Xpander medium (GIBCO, A5019001), supplemented with 500 IU of IL-2 (PeproTech, 200-02), 10 ng/mL of IL-15 (Sino Biological, 10360-HNCE), 10 ng/mL of IL-18 (Sino Biological, 10119-HNCE), and 5% of hAB (Shanghai Hengyuan Biological Technology, HY120-AB). The cells were seeded at a density of 1 × 10⁶ cells/mL, and the medium was half-exchanged once every 2-3 days. The cells were incubated in an incubator at 37 °C and expanded for 14 days for subsequent functional assay. Finally, the harvested NK92 cells and PB-NK cells were characterized by flow cytometry. See Example 8 for the specific procedures and reagents used. The results, as shown in FIG. 20, indicate that the harvested expanded NK92 cells and PB-NK cells both exhibited a viability of 85% or higher, a purity of 85% or higher, and higher expressions of functional receptors. The NKp44 expression in PB-NK cells was lower, about 14%, which may be caused by the dynamic expression of different functional receptors in the NK cells of different peripheral blood sources. Such individual differences are acceptable.
C. *In vitro* killing of GBM by NK cells of different sources in combination with TMZ. The efficacy of iPSC-NK, NK92, and PB-NK cells in combination with TMZ was explored. The *in vitro* killing procedures are shown in Example 9. The effector-to-target E:T ratio = 1:1, and the co-incubation time with GBM tumor cells was set to 8 h. The grouping is shown in Table 6.

**Table 6. Design of study on killing of resistant GBM by NK cells in combination with TMZ**

| Target cell | Group (E:T=1:1), co-incubation for 8 h, replicate=3 | | | | | | | |
|---|---|---|---|---|---|---|---|---|
| Resistant GBM | Negative control | Control | iNK | | NK92 | | PB-NK | |
| | T98G | TMZ+ T98G | iNK+ T98G | iNK+ TMZ+ T98G | NK92+ T98G | NK92+ TMZ+ T98G | PB-NK+T98G | PB-NK+TMZ+ T98G |

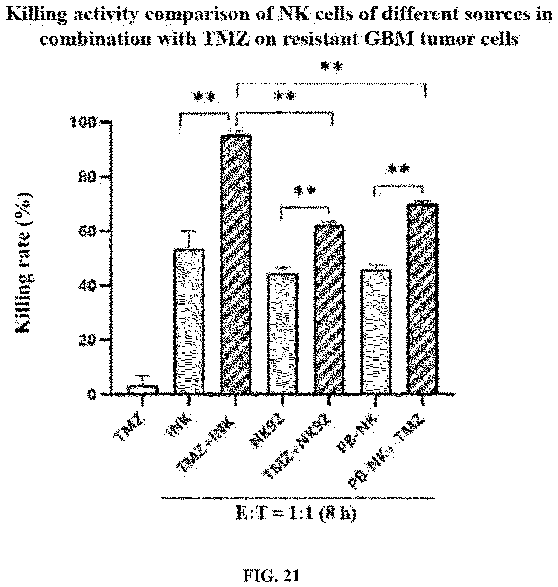

The results, as shown in FIG. 21, indicate that TMZ has substantially no killing effects on resistant GBM (T98G) after 8 h of co-incubation (blank bars). iNK, NK92, and PB-NK cells all exhibited killing effects on target cells (grey bars), with the killing rates reaching 40%-60%. The killing of the target cells was significantly improved in all combination groups of NK cells of various sources with TMZ (black slash bars), with the killing rates being greater than 60%. Further analysis and comparison of the killing activity of iNK, NK92, and PB-NK show that the *in vitro* killing activity of iNK cells in combination with TMZ was superior to that of NK cells of other sources in combination with TMZ. The above results show that the NK cells derived from iPSC differentiation in combination with TMZ have higher tumor killing activity against resistant GBM (T98G) than NK cells of other sources in combination with TMZ.

Although the present disclosure has been disclosed above by preferred embodiments, the embodiments are not intended to limit the present disclosure. Those skilled in the art can make various changes and modifications without departing from the spirit and scope of the present disclosure. Therefore, the scope of the present disclosure should be defined by the claims.

## Claims

1. A kit, comprising a second culture medium, a fourth culture medium, and a fifth culture medium, wherein
the second culture medium is a basal medium comprising a BMP signaling pathway activator; the fourth culture medium is a basal medium comprising at least one, at least two, at least three, or at least four of the following: a BMP signaling pathway activator, a vascular endothelial growth factor, a colony-stimulating factor, and an interleukin;
the fifth culture medium is a basal medium comprising at least one, at least two, at least three, at least four, at least five, or at least six of the following: a BMP signaling pathway activator, a vascular endothelial growth factor, a colony-stimulating factor, an interleukin, a stem cell agonist, and a stem cell expansion agent.

2. The kit according to claim 1, wherein
the stem cell agonist comprises at least one of UM171 and UM729; preferably, the stem cell agonist comprises UM171; and/or
the stem cell expansion agent comprises at least one of SR1 and PD98059; preferably, the stem cell expansion agent comprises SR1.

3. The kit according to claim 1 or 2, wherein
the kit further comprises a third culture medium; the third culture medium is a basal medium comprising at least one, at least two, or at least three of the following: a BMP signaling pathway activator, a growth factor, and a TGFβ/ALK inhibitor.

4. The kit according to any one of claims 1-3, wherein
the kit further comprises a first culture medium; the first culture medium is a basal medium comprising a ROCK inhibitor.

5. The kit according to any one of claims 1-4, wherein
the second culture medium is a basal medium comprising a BMP signaling pathway activator and a GSK3 inhibitor.

6. Use of the kit according to any one of claims 1-5 in preparing an embryoid body, preparing a mesodermal cell, preparing a hemogenic endothelial cell, preparing a natural killer (NK) cell, preparing a product for inducing the differentiation of a stem cell into an embryoid body, preparing a product for inducing the differentiation of a stem cell into a mesodermal cell, preparing a product for inducing the differentiation of a stem cell into a hemogenic endothelial cell, or preparing a product for inducing the differentiation of a stem cell into an NK cell.

7. A method for inducing the differentiation of a stem cell into a natural killer (NK) cell, comprising: using the kit according to any one of claims 1-5.

8. The method according to claim 7, wherein
the method comprises:
step (1), culturing a stem cell to give an embryoid body;
step (2), subjecting the embryoid body to mesoderm differentiation culture to give a mesodermal cell;
step (3), subjecting the mesodermal cell to hemogenic endothelium differentiation culture to give a hemogenic endothelial cell;
step (4), culturing the hemogenic endothelial cell in the fourth culture medium and the fifth culture medium of the kit according to any one of claims 1-5 to give the NK cell;
preferably, the culture in step (1) is performed in the first culture medium of the kit according to claim 4;
preferably, the culture in step (2) is performed in the second culture medium of the kit according to claim 5;
preferably, the culture in step (3) is performed in the third culture medium of the kit according to claim 3;
preferably, the time for the culture in step (1) is 12-36 h;
preferably, the time for the culture in step (2) is 24-72 h;
preferably, the time for the culture in step (3) is 48-144 h;
preferably, in step (4), culturing the hemogenic endothelial cell in the fourth culture medium and the fifth culture medium of the kit according to any one of claims 1-5 comprises sequentially culturing the hemogenic endothelial cell in the fourth culture medium and the fifth culture medium of the kit according to any one of claims 1-5;
preferably, the culture time of the hemogenic endothelial cell in the fourth culture medium is 96-192 h;
preferably, the culture time of the hemogenic endothelial cell in the fifth culture medium is 144-432 h.

9. A natural killer (NK) cell, prepared using the kit according to any one of claims 1-5 or the method according to claim 7 or 8, wherein
preferably, the content of CD3⁻/CD45⁺/CD56⁺ cells in the NK cells is 90% or greater;
preferably, the content of CD3⁻/CD56⁺/Nkp44⁺ cells in the NK cells is 90% or greater;
preferably, the content of CD3⁻/CD56⁺/Nkp30⁺ cells in the NK cells is 85% or greater.

10. A pharmaceutical composition, comprising the NK cell according to claim 9, wherein preferably, the pharmaceutical composition further comprises a pharmaceutically acceptable carrier.

11. The pharmaceutical composition according to claim 10, further comprising an anti-tumor drug.

12. Use of the NK cell according to claim 9 or the pharmaceutical composition according to claim 10 or 11 in preparing a medicament for preventing and/or treating a tumor.

13. A pharmaceutical combination for use in treating glioblastoma, comprising an active ingredient, wherein the active ingredient comprises a natural killer (NK) cell and a chemotherapeutic agent; the chemotherapeutic agent comprises temozolomide (TMZ).

14. The pharmaceutical combination according to claim 13, wherein the glioblastoma is TMZ-resistant.

15. The pharmaceutical combination according to claim 13 or 14, wherein the NK cell is an unmodified NK cell.

16. The pharmaceutical combination according to any one of claims 13-15, wherein the NK cell is an induced pluripotent stem cell (iPSC)-derived NK cell.

17. The pharmaceutical combination according to any one of claims 13-16, wherein the NK cell is prepared using the kit according to any one of claims 1-5 or the method according to claim 7 or 8.

18. The pharmaceutical combination according to any one of claims 13-17, further comprising an additional chemotherapeutic agent selected from an anti-cancer agent, a metabolic antagonist, a DNA demethylating agent, a plant-derived anti-tumor agent, an alkylating agent, an antimetabolite, an anti-cancer antibiotic, a topoisomerase inhibitor, a mitotic inhibitor, a differentiation agent, and a hormonal therapeutic agent.

19. The pharmaceutical combination according to claim 18, wherein the additional chemotherapeutic agent is selected from carmustine, lomustine, vorinostat, mebendazole, imatinib mesylate, AG119, and any therapeutic derivative thereof.

20. The pharmaceutical combination according to any one of claims 13-19, wherein the active ingredient does not comprise trimetrexate.

21. The pharmaceutical combination according to any one of claims 13-20, wherein the active ingredient does not comprise an immune checkpoint inhibitor.

22. The pharmaceutical combination according to any one of claims 13-21, wherein the pharmaceutical combination is a kit.

23. Use of the pharmaceutical combination according to any one of claims 13-22 in preparing a medicament for treating glioblastoma.

24. The use according to any one of claims 13-23, wherein
the chemotherapeutic agent is administered before, after, or concurrently with the administration of the NK cell.

25. The use according to any one of claims 13-24, wherein the chemotherapeutic agent is administered 8 to 72 h before, 8 to 72 h after, or 8 to 72 h from the administration of the NK cell.
